# EUROPEAN PATENT SPECIFICATION

(11) **EP 0 842 610 B1**
(45) Date of publication and mention of the grant of the patent: **15.01.2003**
(21) Application number: 97924245.0
(22) Date of filing: 28.05.1997
(51) Int. Cl.: A23K 1/00, A23K 1/16, A61K 35/78, A61K 35/84

(54) **VEGETABLE FOOD SUPPLEMENT FOR DEODORISING FECES AND URINE**
PFLANZLICHER NAHRUNGSZUSATZ ZUM DESODORIEREN VON STUHL UND URIN
ADDITIF ALIMENTAIRE VEGETAL REDUISANT L'ODEUR DES MATIERES FECALES ET DE L'URINE

(30) Priority: 28.05.1996 JP 15759096
(43) Date of publication of application: 20.05.1998
(73) Proprietor: Toyo Hakko Co., Ltd., Obu-shi, Aichi 474 (JP)
(72) Inventor: KIMURA, Akihiko, Obu-shi Aichi 474 (JP); ISHIKAWA, Naoto, Obu-shi Aichi 474 (JP); TAKADA, Atsushi, Obu-shi Aichi 474 (JP); OKADA, Toshitaka, Obu-shi Aichi 474 (JP)
(74) Representative: Kearney, Kevin David Nicholas
(86) International application number: JP9701816
(87) International publication number: WO97045023

(56) References cited:
- JP-A- 3 119 974
- JP-A- 3 147 750
- JP-A- 5 192 087
- JP-A- 5 336 895
- JP-A- 56 092 745
- JP-A- 63 000 266

## Description

### TECHNICAL FIELD

The present invention relates to a plant-type feces and urine deodorant that, when orally administered to a living body, has an effect of reducing odor of feces and urine discharged from the living body, and to a plant-type feces and urine deodorant that has an effect of reducing urinary concrement as well as the odor reducing effect. The plant-type feces and urine deodorant of the invention is for use to pet animals, such as dogs, cats, farm animals, such as chickens, pigs, cows, and also for use to humans.

### BACKGROUND ART

To eliminate unpleasant odor produced by pets, such as cats, 1) so-called cat litter, 2) deodorizing or reodorizing spray, have conventionally been used. Furthermore, 3) urine odor eliminating agents containing phytic acid or a salt thereof as an effective component, are also known (Japanese patent application laid-open No. Hei 1-195860, and Japanese patent application laid-open No. Hei 1-275522). Further, 4) plant-type deodorants containing as a main component a predetermined dry powder of leaves or the like of Umbelliferae plants, such as celery, carrot, are also known (Japanese patent application laid-open No. Sho 63-262156).

However, the cat litter 1) mentioned above is used to adsorb and eliminate odor from discharged feces and urine. That is, feces and urine are discharged with their odors uneliminated. Therefore, the deodorizing effect of cat litter cannot be achieved unless discharge is performed on the litter. The cat litter also has handling difficulty due to powdering. In use of the deodorizing or reodorizing spray 2) mentioned above, it is necessary to perform a spraying action. In addition, the spray merely deodorizes or reodorizes feces and urine after they have been discharged, similar to the cat litter.

The urine deodorizing agents 3) are agents that are fed to cats before discharge of urine, in order to eliminate urine odor. However, the agents are not for eliminating feces odor. Furthermore, since the urine deodorizing agents are not fermentation products that have been fermented, there are cases where thenutritional balance or the safety of the agents is not sufficient for administration to living bodies. The deodorants 4) cannot be said to have sufficiently concentrated effective components, due to the direct powdering of specific parts of plants. Moreover, the deodorants 4) cannot be said to be sufficient in deodorizing effect.

Japanese patent application laid-open No. Hei 5-192087 discloses a fermentation health feed which contains a fermentation liquid and a tree component extract liquid and which prevents production of unpleasant odor of feces and urine by feeding it to pets or farm animals. Japanese patent application laid-open No. Hei 5-336895 discloses a plant-type deodorant which contains a fermentation liquid and an extract liquid of carrot leaves and which eliminates the odor of feces, urine by a method wherein it is fed to pets, or wherein it is sprayed it to an object to be deodorized. However, neither Japanese patent application laid-open No. Hei 5-192087 nor Japanese patent application laid-open No. Hei 5-336895 describes the deodorizing effect on feces or urine discharged from human bodies when the fermentation health feed or the plant-type deodorant has been taken by the human bodies.

The present invention is accomplished in view of the aforementioned circumstances. It is an object of the invention to provide a plant-type feces and urine deodorant which, when orally administered to a living body, has an effect of reducing the odor of feces and urine discharged from the living body, and which is safe, and which also has an effect of reducing urinary concrement.

### DISCLOSURE OF THE INVENTION

The plant-type feces and urine deodorant of the invention described in claim 1 is characterized by: containing (1) a fermentation liquid or a vaporization residue thereof (hereinafter, either one of these is referred to as "fermentation liquid"), the fermentation liquid being produced by inoculating Bacillus subtilis or Bacillus natto into a liquid medium which contains rice bran, soybean, a carbon source and water, and which has been adjusted to pH 7.5-10 by an alkaline agent, and by culturing it and filtering it, (2) a tree component extract liquid made using at least one of cedar, cypress and pine as a raw material, or a vaporization residue of the tree component extract liquid (hereinafter, either one of these is referred to as "tree component extract liquid"), and (3) at least one selected from a tea leaf extract liquid or a vaporization residue thereof, an Umbelliferae plant extract liquid or a vaporization residue thereof, or a mushroom extract liquid or a vaporization residue thereof (hereinafter, either an extract liquid or a vaporization residue thereof of each species is referred to as "tea leaf extract liquid", "Umbelliferae plant extract liquid" or "mushroom extract liquid", respectively) ; and having an effect of reducing odor of feces and urine through oral administration.

The plant-type feces and urine deodorant of the invention described in claim 5 further has an effect of reducing urinary concrement, while employing a fermentation liquid, a tree component extract liquid, and a tea leaf extract liquid, an Umbelliferae plant extract liquid, and a mushroom extract liquid that are substantially the same as in the invention described in claim 1.

The "rice bran" means rice germ, defatted rice germ, rice bran, defatted rice bran. The "soybean" means defatted soybean, parched soy flour, soybean flour, soybean cake, hydrolysates of these materials. It is preferred that the proportion of the amount of the rice bran and the amount of the soybean be determined such that the amount of the soybean is 10-500 parts by weight (particularly preferably, 10-20 parts by weight) relative to 100 parts by weight of the rice bran. If the proportion of the amount of the soybean is less than 10 parts by weight, the amount of peptides becomes inconveniently small. If the proportion of the amount of the soybean exceeds 500 parts by weight, the rate of decomposition of the soybean decreases. As the "carbon source", normally employed carbon sources (for example, one or more species of glucose, dextrin, lactose, starch) may be used.

The medium used in the invention described in claim 1 and the invention described in claim 5 is "a liquid medium" containing water. Normally, the medium further contains a phosphorus compound such as phosphoric acid salts, phytic acid. It is preferable that phytic acid be used in the form of a sodium salt or a calcium salt. Normally, the fermentation conditions in the invention described in claim 1 and the invention described in claim 5 are that the pH value is 7.5-10 (particularly, about 9), and the culturing temperature is about 40-45°C. Such alkaline culture is essential because alkaline culture produces larger amounts of effective components for the feces-and-urine deodorizing and reodorizing effect and the urinary concrement reducing effect. To provide such an alkaline culture, sodium hydrogen carbonate may be used as an alkaline agent.

Further, it is preferable that, in the liquid medium, the concentrations of the rice bran, the soybean, and the carbon source be respectively 5-15% by weight relative to the total medium containing the rice bran, the soybean, the carbon source and water. If the concentration of any of these materials is less than 5% by weight, the concentration is excessively low so that the production efficiency deteriorates. If it exceeds 15% by weight, ventilation-stirring efficiency inconveniently decreases. Because of the liquid culture, culturing can be achieved by ventilation stirring.

As a material of the medium, alkali protease may be used in order to accelerate decomposition of soybean peptides. The amount of alkali protease added is normally 0.001-1% by weight. If the amount of alkali protease added is less than 0.001% by weight, the decomposition efficiency decreases. If the amount added exceeds 1% by weight, the decomposition efficiency will not improve while the cost increases.

The "fermentation liquid" may be a liquid obtained by mere filtrate of a culture fermentation liquid used for culture, or a liquid obtained by post-treatment of the filtrate, such as decolorization thereof. Furthermore, the liquid may be concentrated into a concentration liquid, or may be purified by distillation. Further, the fermentation liquid may be a vaporization residue obtained by drying the "fermentation liquid" by a method such as spray-drying, heating, vacuum-drying, or may be dried solid.

The "tree component extract liquid or the like" can be obtained by, for example, dry distillation, steam distillation, solvent extraction of plant including cedar, cypress and/or pine, or removing solvent therefrom. As the plant, conifers are particularly preferable. The tree component extract liquid may be a liquid directly obtained through treatment, or a concentrated liquid thereof, or a diluted liquid using water. The vaporization residue of the tree component extract liquid is obtained by drying the tree component extract liquid using a method, such as spray-drying. The vaporization residue thereof may be dried solid. If the tree component extract liquid has an organic content of about 6% by weight, that is, a moisture content of 94% by weight, it is preferable that the amount of the tree component extract liquid compounded be 0.1-20 parts by weight relative to 100 parts by weight of the fermentation liquid (the vaporization residue concentration being 6% by weight). If the amount compounded is less than 0.1% by weight, the feces and urine deodorizing and reodorizing effect and the urinary concrement reducing effect may be insufficient in some cases. If the amount compounded exceeds 20 parts by weight, the effect tends to become saturated. As for the weight ratio of vaporization residue, it is preferable that the amount of the vaporization residue in the tree component extract liquid be about 0.001-0.2 part by weight (preferably, 0.05-0.1 part by weight) relative to 1 part by weight of the vaporization residue in the fermentation liquid.

The "tea leaf extract liquid" means anything that is conceived as "tea", that is, any liquid for people to drink produced by dipping leaves into hot water, or any material obtained by removing solvent or the like from the liquid. Examples of the "tea" are green tea, tea leaves for black tea, tea leaves for oolong tea, rooibos tea (scientific name: Aspalathus linearis), hawthorn, fleawort. Among these, green tea, rooibos tea or hawthorn is particularly preferably used. Tea leaves are extracted using a solvent containing at least one of water and ethanol, and the extract is dried to obtain the tea leaf extract liquid.

The "Umbelliferae plant extract liquid" means an extract liquid obtained from at least one species of plant selected from Umbelliferae plants, such as carrot, celery, parsley, Oenanthe, Cryptotaenia japonica, anise or Foeniculum, Angerica, Ligusticum, or a vaporization residue of the extract liquid. The extract liquid may be obtained from any part of a plant indicated above, such as leaves, stems, roots, and, further, may be in the form of a mixture thereof. In particular, it is preferable that the Umbelliferae plant extract liquid or the like contain an extract liquid from leaves of a plant indicated above since leaves contain large amounts of effective components. Furthermore, the extract liquid may be provided in a form wherein it is compounded into, for example, a beverage. A vaporization residue obtained by drying the extract liquid may be used in substantially the same manner.

The "mushroom extract liquid" means extract liquids obtained from mushrooms using solvents containing at least one of water and ethanol, and vaporization residues thereof. The "mushrooms" means general mushrooms, such as agaric, Lentinus edodes, Grifola. Particularly, it is prefereable to use a mushroom having a scientific name of Agaricus Bisporus (so-called agaric).

The methods of preparing the tea leaf extract liquid, the Umbelliferae plant extract liquid and the mushroom extract liquid are not particularly limited as long as the object of the present invention can be achieved. For example, a method in which leaves of a certain Umbelliferae plant are boiled and essence is extracted, and then the pressing and filtration thereof is performed, may be cited. The extract liquid may also be concentrated, sterilized or subjected to other treatment, if necessary. The extract liquid may also be dried by a method, such as spray-drying, heating, vacuum-drying, so as to form a vaporization residue or a dry solid for use. The tea leaves and the Umbelliferae plant used in the invention are preferably fresh ones just collected. If a long time elapses after collection, the plant leaves or the like lose freshness, flavor, and may cause deterioration of the deodorizing or reodorizing effect or the urinary concrement reducing effect.

In the invention described in claim 1 and the invention described in claim 5, it is preferred that the at least one selected from the tea leaf extract liquid, the Umbelliferae plant extract liquid and the mushroom extract liquid (hereinafter, referred to as "plant extract liquid") be compounded so that the amount thereof becomes 10-1000 parts by weight, particularly, 20-500 parts by weight, more preferably, 30-350 parts by weight, relative to 100 parts by weight of the vaporization residue of the fermentation liquid. Within this range of compounding proportion, the two liquids will synergistically produce their effects. If the plant extract liquid is formed of any one selected from the tea leaf extract liquid, the Umbelliferae plant extract liquid and the mushroom extract liquid, it is preferable that the amount of the vaporization residue of the plant extract liquid be 10-1000 parts by weight, 20-500 parts by weight, more preferably, 30-350 parts by weight, relative to 100 parts by weight of the vaporization residue of the fermentation liquid, for substantially the same reasons as stated above.

If the plant extract liquid is formed of the tea leaf extract liquid and the Umbelliferae plant extract liquid and/or the mushroom extract liquid, it is preferable that these extract liquids be compounded so that the amount of the Umbelliferae plant extract liquid and/or the mushroom extract liquid is 10-1000 parts by weight (particularly, 20-250 parts by weight) relative to 100 parts by weight of the vaporization residue of the tea leaf extract liquid.

As for the nature or state of the plant-type feces and urine deodorant of the invention, the plant-type feces and urine deodorant may be a mixture liquid containing the components (1)-(3), or a concentration or a dilution with water, or may be mixed into other drinks. Furthermore, it may be a vaporization residue of the mixture liquid. For example, it is possible to use a powdered product obtained by drying the mixture solution through spray-drying. Further, it is possible to prepare pills, tablets, capsules containing the vaporization residue, or foods containing the vaporization residue.

According to the invention, it is also possible to compound dietary fiber, such as vegetable fiber, in addition to the components (1)-(3). Thereby, the plant-type feces and urine deodorant of the invention will not be excessively absorbed by a stomach but will unfailingly reach the intestine, so that the deodorizing effect and the urinary concrement reducing effect will be enhanced. The amount of dietary fiber compounded is preferably 1-100 parts by weight (particularly, 5-80 parts by weight) relative to 100 parts by weight of the vaporization residues of the components (1)-(3).

It is also possible to add vitamins, in order to improve the nutritional balance and enhance the deodorizing effect and the urinary concrement reducing effect. In particular, addition of vitamin C can further enhance the deodorizing effect and the urinary concrement reducing effect. The amount of vitamin C added is preferably 0.05-100 parts by weight (particularly, 0.1-50 parts by weight) relative to 100 parts by weight of the vaporization residues of the components (1)-(3). In addition, it is possible to add an extract of a fruit such as citrus. Since such fruit extracts contain large amounts of vitamin C, addition of such an extract will enhance the deodorizing effect and the urinary concrement reducing effect. Further, due to the flavor of a fruit extract added, the deodorant will advantageously become easy to take in.

In the plant-type feces and urine deodorant of the invention described in claim 1 and the invention described in claim 5, the tree component extract liquid produced using at least one of cedar, cypress and pine as a raw material, and a predetermined plant extract liquid are contained in a predetermined fermentation liquid. When taken into a living body, the plant-type feces and urine deodorant produces the effect of remarkably reducing the odor of feces and urine discharged from the living body that has taken the deodorant and the effect of reducing urinary concrement. Since feces and urine are already deodorized when discharged from the living body, the plant-type feces and urine deodorant of the invention is excellent in prevention of diffusion of odor to the outside. Furthermore, since the plant-type feces and urine deodorant is made from a material obtained through natural fermentation of completely natural materials and from extracts from natural materials, the plant-type feces and urine deodorant of the invention is safe to living bodies. Further, since the plant-type feces and urine deodorant is made from rice bran and a soybean product, which are useful as health foods, and contains useful nitrogen compounds (amino acids, ash, phosphorus compounds, the plant-type feces and urine deodorant has good nutritional balance. In comparison with a case where plants are directly used, the plant-type feces and urine deodorant of the invention employs extract liquids wherein unuseful solids are removed, so that it has higher effective component concentrations and better quality.

Although an agent containing the components (1) and (2) and not containing the component (3) of the components of the plant-type feces and urine deodorant of the invention has an effect of, when orally administered to a human body, reducing the odor of feces and urine discharged from the human body and an effect of reducing urinary concrement, addition of the component (3) further enhances the odor reducing effect and the urinary concrement reducing effect.

### BRIEF DESCRIPTION OF THE DRAWINGS

Fig. 1 is a graph indicating the deodorizing effect in Example 1. Fig. 2 is a graph indicating the deodorizing effect in Example 1, wherein the odor strength of the control group is determined as 100%.

Fig. 3 is a graph indicating the deodorizing effects of deodorants A-D relative to that of the control group in Example 2. Fig. 4 is a graph indicating the deodorizing effects of deodorants E-G in Example 2.

Fig. 5 is a graph indicating the deodorizing effects of deodorants H, I and J in Example 3. Fig. 6 is a graph indicating the deodorizing effects of deodorants K and L in Example 3.

Fig. 7 is a graph indicating the urinary concrement reducing effects of mixtures B and C in Example 4.

### BEST MODES FOR CARRYING OUT THE INVENTION

Examples of the invention will be described hereinafter.

### [Example 1]

In Example 1, the human feces and urine-deodorizing effect of a plant-type feces and urine deodorant according to the invention described in claim 1 in oral administration of the deodorant was investigated.

### (1) Production of Fermentation Liquid and Preparation of Plant-type Feces and Urine Deodorant (Mixture Liquid)

### 1) Production of Fermentation Liquid

A fermentation liquid was produced by a method described below.

First, a liquid medium made of 30 kg of defatted rice bran, 3 kg of soybean cake (or defatted soybean), 5 kg of bittern and 500 kg of water was prepared. The pH of the liquid medium was about 9. After the medium was disinfected at 121°C for 30 minutes, the medium was cooled and then seeded with 0.05 kg of Bacillus natto (by Naruse Hakkou Kagaku Kenkyuusho). Culture was performed at 40-45°C for about 48 hours while the medium was being ventilated and stirred.

Subsequently, the culture was subjected to press-filtration followed by treatment with active carbon and pearlite for deodorization and decolorization, thereby obtaining a fermentation liquid. As the active carbon, various types may be used, for example, powder active carbons (active carbon S, active carbon K), particle active carbons (native carbon SG). As the pearlite, a pearlite commercially named as "Pearlite No. 4180" (by Daika Line Orient Kabushiki Gaisha) was used. The obtained fermentation liquid was a colorless or light yellow transparent liquid having a fermentation flavor and a water content of 94% (according to a normal pressure heat-drying method). In the liquid, none of general living microorganisms, Escherichia coil, fungi, yeast, arsenic and heavy metals was detected.

### 2) Preparation of Plant-type Feces and Urine Deodorant (Mixture Liquid)

A mixture liquid was obtained by compounding a tree component extract liquid having a vaporization residue concentration of 1% by weight and a tea leaf (green tea) extract liquid having a vaporization residue concentration of 47% by weight into 100 parts by weight of the fermentation liquid (vaporization residue concentration: 6.0% by weight). The compounding proportions were predetermined such that 2 parts by weight of the vaporization residue in the tree component extract liquid and 60 parts by weight of the vaporization residue in the tea leaf extract liquid were present relative to 100 parts by weight of the vaporization residue in the fermentation liquid.

The tree component extract liquid used in this example was obtained by dry-distilling approximately equal amounts of cedar, cypress and pine chips at 120-180°C for several tens of hours, and then distilling the material at a temperature of about 100-120°C (at normal pressure or a reduced pressure) to remove mainly tar contents, and, if necessary, diluting the material with water. An essential oil thereof obtained by steam distillation may also be used. The pH of the tree component extract liquid was 6-7, and the color thereof was colorless or light yellow or light brown. The tree component extract liquid had a slight flavor. The tea leaf extract liquid used in this example was obtained by extracting green tea leaves with a mixed solvent of water and ethanol. The specific weight of the tea leaf extract liquid was about 1.08. As a material for the tea leaf extract liquid, green tea leaves of "Camellia thea" in scientific name was used.

### 3) Formation of Tablets

An excipient made of glucose, a sucrose ester, citric acid and a lemon flavor was added to the mixture liquid. The proportion of the amount of the excipient added was 2 parts by weight of the excipient relative to 1 part by weight of the vaporization residue (hereinafter, referred to as "effective deodorizing component") of the mixture liquid. The mixture was powdered by spray-drying, and then formed into tablets of 0.3g/tablet, which were used as a test sample. That is, the test sample contained 0.1 g of the effective deodorizing component per tablet.

### (2) Method and Result of Performance Evaluation

### 1) Test Method

Student house-living people receiving the same diet were used as subjects. 30 student house-living people were divided into two groups (determined as group 1 and group 2) of 15 people each.

### 1) First Course (10 Days)

In the first course, group 1 and group 2 were determined as a control group and a test group, respectively. In addition to meals, 3 baked garlic pieces of about 5 g each were given daily for 10 consecutive days, in both group 1 and group 2. Three test sample tablets per day (that is, 0.3 g of the effective deodorizing component per day) were given for the 10 consecutive days, only in group 2.

### 2) Non-test Period (7 Days)

The seven days following the first course was determined as a non-test period during which neither baked garlic nor the test sample was given to either group 1 or group 2.

### Second Course (10 Days)

Subsequently, the second course was conducted with group 1 determined as a test group and group 2 determined as a control group, that is, the group assignment opposite to that in the first course. In both group 1 and group 2, 3 baked garlic pieces of about 5 g each were given daily for 10 consecutive days, besides meals. Three test sample tablets were given daily for the 10 consecutive days, in only group 1. The meals given during these periods included no food that is likely to produce odor, such as garlic. The student house-living people were given the same contents of meals at the same hours. All the student house-living people were healthy and discharged normal stool before the test and during the test.

Evaluation of odor was conducted in the manner of sensory evaluation performed by each person at the test. Evaluation references were as follows: "0" for no odor; "1" for odor slightly detectable; "2" for odor to such an extent that its odor type was detectable; "3" for odor easily detectable; "4" for strong odor; and "5" for very strong odor. Based on this, points were given. The points of odor were determined as follows. The case "0" of no odor was assigned with point 1, and the case "5" of strong odor was assigned with 6, and the other cases were assigned with the corresponding values, that is, values obtained by adding 1 to the values of the odor evaluation cases.

### 2) Evaluation Results

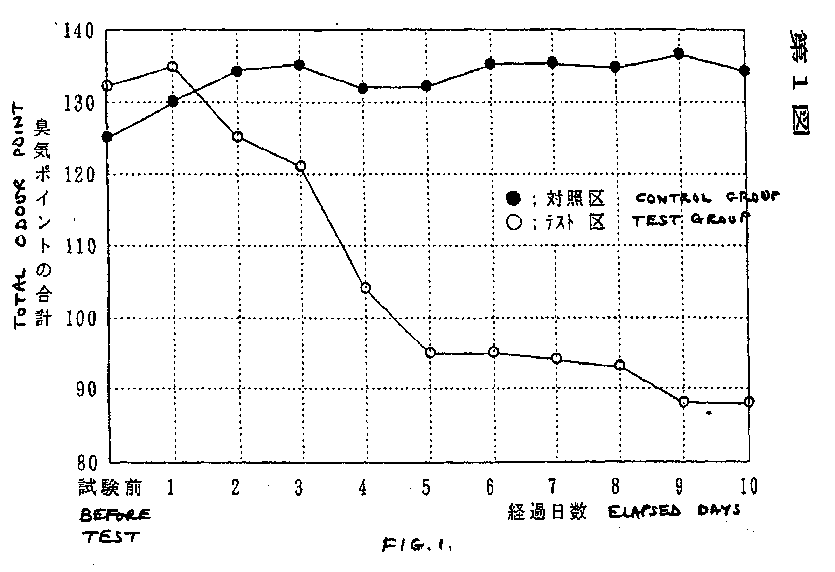

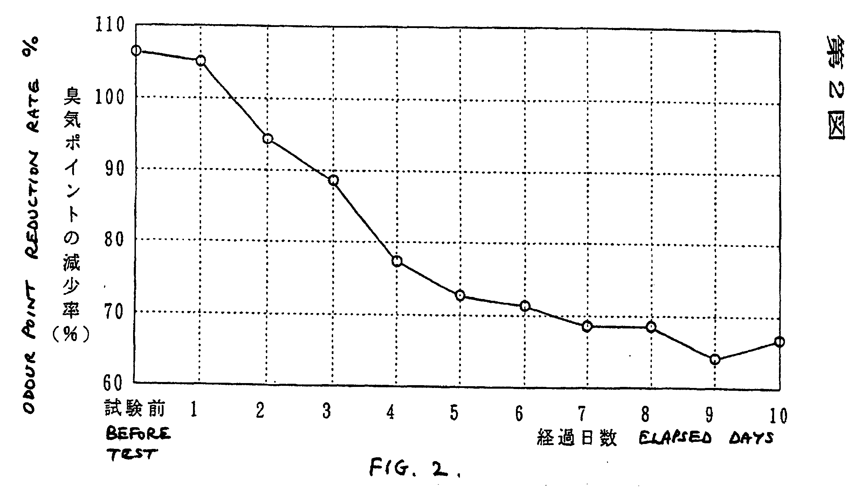

Test results of the control groups (30 people) of the first course and the second course are shown in Table 1. Test results of the test groups (30 people) of the first course and the second course are shown in Table 2. The total points in the results shown in Table 1 are presented in a graph shown in Fig. 1. The reduction rate of odor points of the test groups relative to the odor points of the control groups which are determined as 100% are indicated in Fig. 2.

As can be seen from Table 1, Table 2, Fig. 1 and Fig. 2, in the test groups, which were given a daily dose of three test sample tablets containing 0.1 g of the effective deodorizing component per tablet, an odor reduction started to show 2-3 days after the test started, and the odor strength became about 70% or less relative to that of the control groups from the fourth or fifth day on. Thus, a odor reducing effect was clearly observed. In addition, as indicated in Table 1 and Table 2, while the proportion of subjects who reported "strong odor" or "very strong odor" (that is, odor evaluation references of 4 or higher) was about 50% in the control groups throughout the test period, the proportion of such subjects in the test groups decreased to 30% three days after the test started, and became 0% from the sixth day on. These results also indicate that the test sample had a clear deodorizing effect.

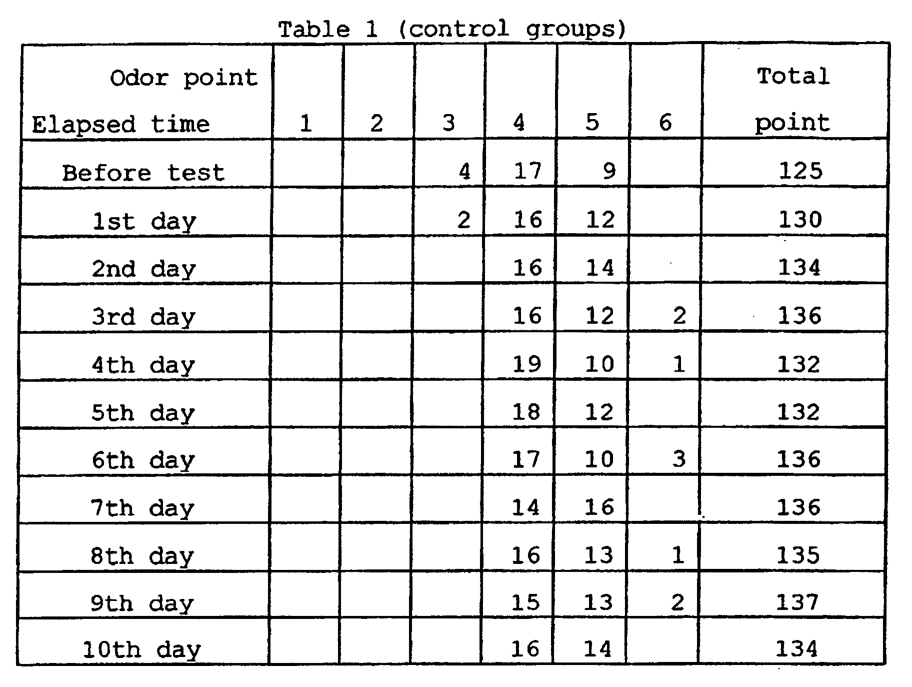

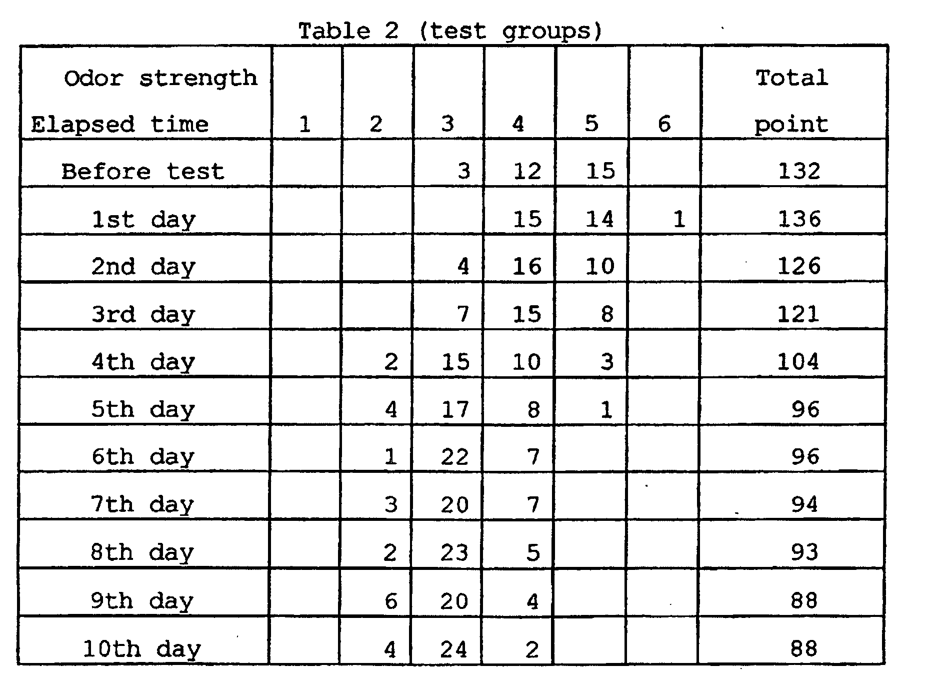

### [Example 2]

In Example 2, the effect of combined use of a fermentation liquid, i.e., the component (1), and a tree component extract liquid, i.e., the component (2), and the effect of a plant-type feces and urine deodorant according to the invention further employing a tea leaf extract liquid, i.e., the component (3), in addition to the components (1) and (2), were checked.

### [1] Effect of Combined Use of Fermentation Liquid, Component (1), and Tree Component Extract Liquid, Component (2)

### (1) Preparation of Deodorant

Deodorants were prepared in substantially the same manner as in Example 1.
1) Deodorant A: wherein only the vaporization residue of the fermentation liquid as used in Example 1 was used.
2) Deodorant B: wherein compounding was performed such that that 0.1 part by weight of the vaporization residue of the tree component extract liquid was contained relative to 100 parts by weight of the vaporization residue of the fermentation liquid as used in Example 1.
3) Deodorant C: based on deodorant B, wherein the vaporization residue of the tree component extract liquid was present in an amount of 2 parts by weight.
4) Deodorant D: based on deodorant B, wherein the vaporization residue of the tree component extract liquid was present in an amount of 20 parts by weight.

### (2) Formation of Tablets

As in Example 1, tablets of 0.3 g/tablet, containing 0.1 g of the effective deodorizing component per tablet, were formed and used as test samples.

### (3) Method and Result of Test

Student house-living people receiving the same diet were used as subjects. 50 student house-living people were divided into five groups (determined as groups 1)-5)) of 10 people each. The groups are differently treated as follows:
Group 1): control group
Group 2): took 3 deodorant A-containing tablets/day.
Group 3): took 3 deodorant B-containing tablets/day.
Group 4): took 3 deodorant C-containing tablets/day.
Group 5): took 3 deodorant D-containing tablets/day.

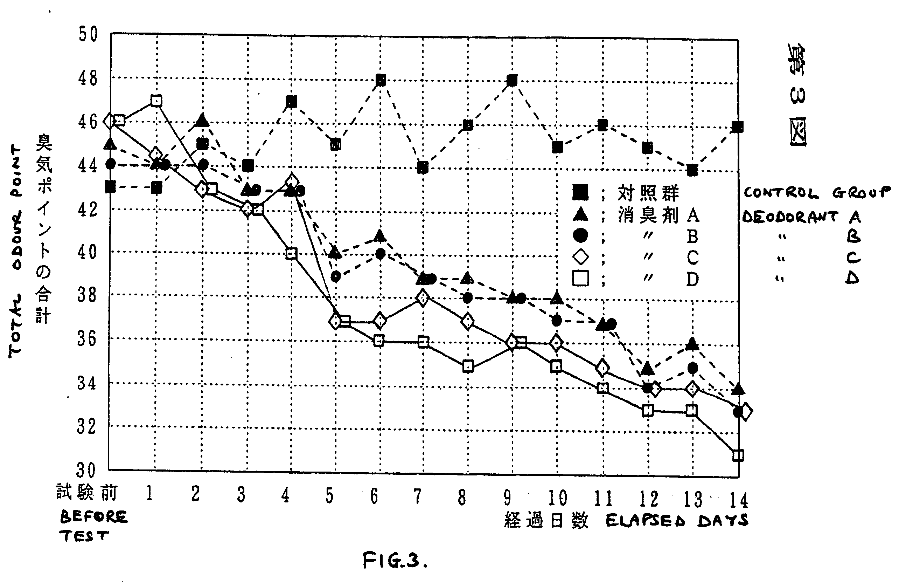

The test was performed for 14 days. In each group, 3 baked garlic pieces of about 5 g each were daily given for 14 consecutive days, in addition to meals. Evaluation of odor was conducted as in Example 1. That is, each person at the test performed sensory evaluation of odor. Based on evaluation references substantially the same as those used in Example 1, points were given. Results are shown in Tables 3-7. The meals given during the period included no food that is likely to produce odor, such as garlic. The student house-living people were given the same contents of meals at the same hours. All the student house-living people were healthy and discharged normal stool before the test and during the test. The total odor points in the results shown in Tables 3-7 are presented in a graph shown in Fig. 3.

As can be seen from Tables 3-7 and Fig. 3, no odor reduction occurred in the control group. On the other hand, in the groups that received the deodorants, odor started to decrease on the fourth or fifth day, and final reductions of about 25-30% in odor strength were observed. Although deodorants A-D did not show a significant difference in deodorizing effect, deodorants C and D, containing 2 parts by weight and 20 parts by weight of the vaporization residue of the tree component extract liquid, produced apparently greater deodorizing effects from the fifth day on than deodorant B, which contained only the vaporization residue of the fermentation liquid. Thus, an advantage of the combined use of the tree component extract liquid was observed.

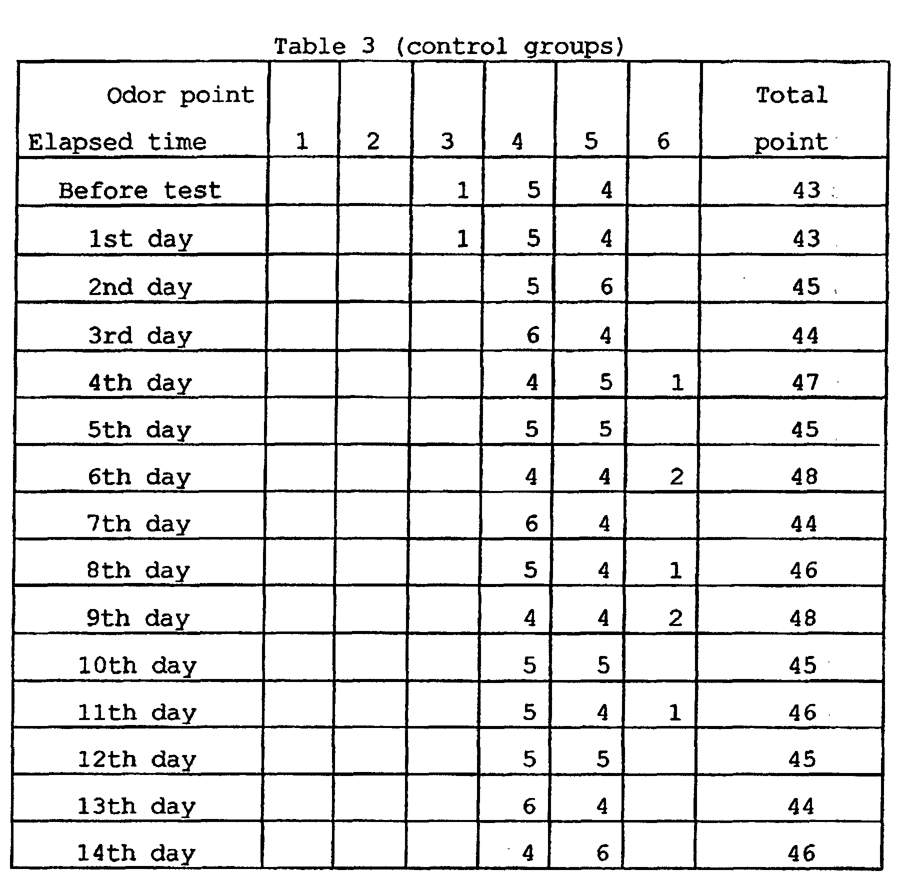

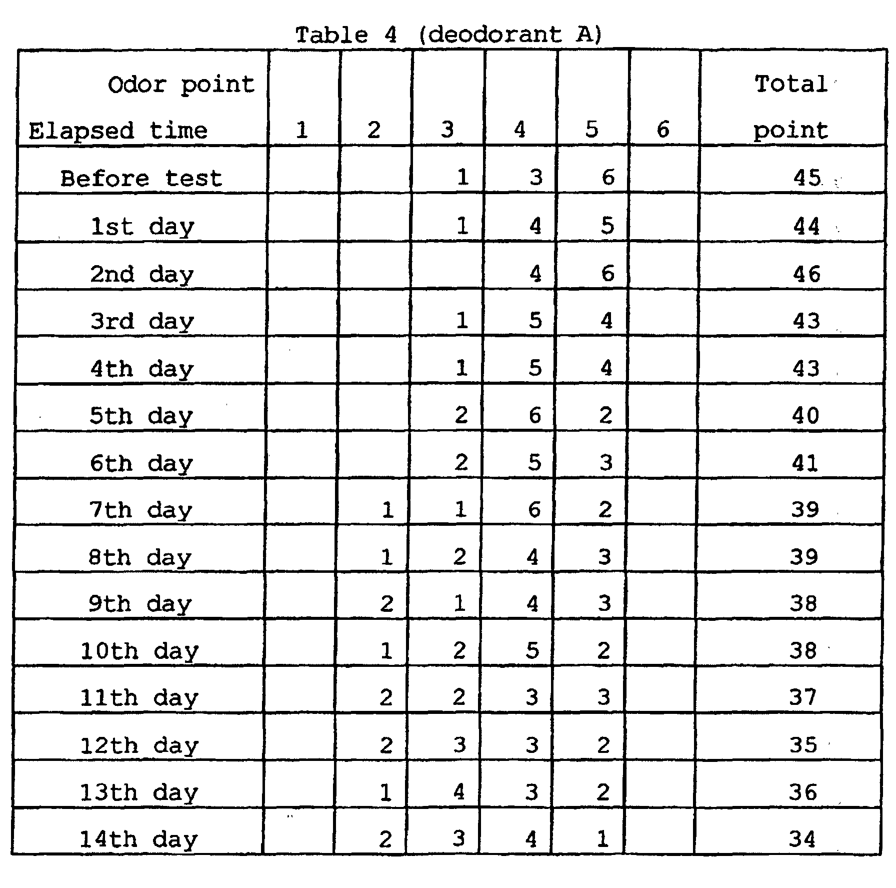

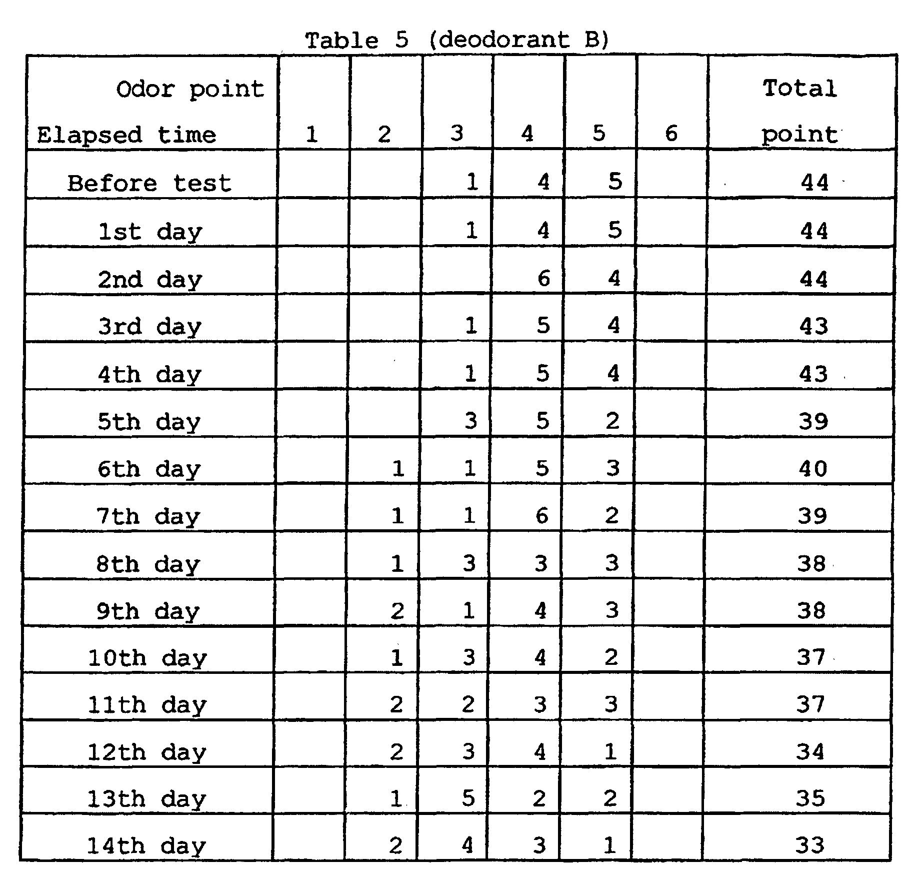

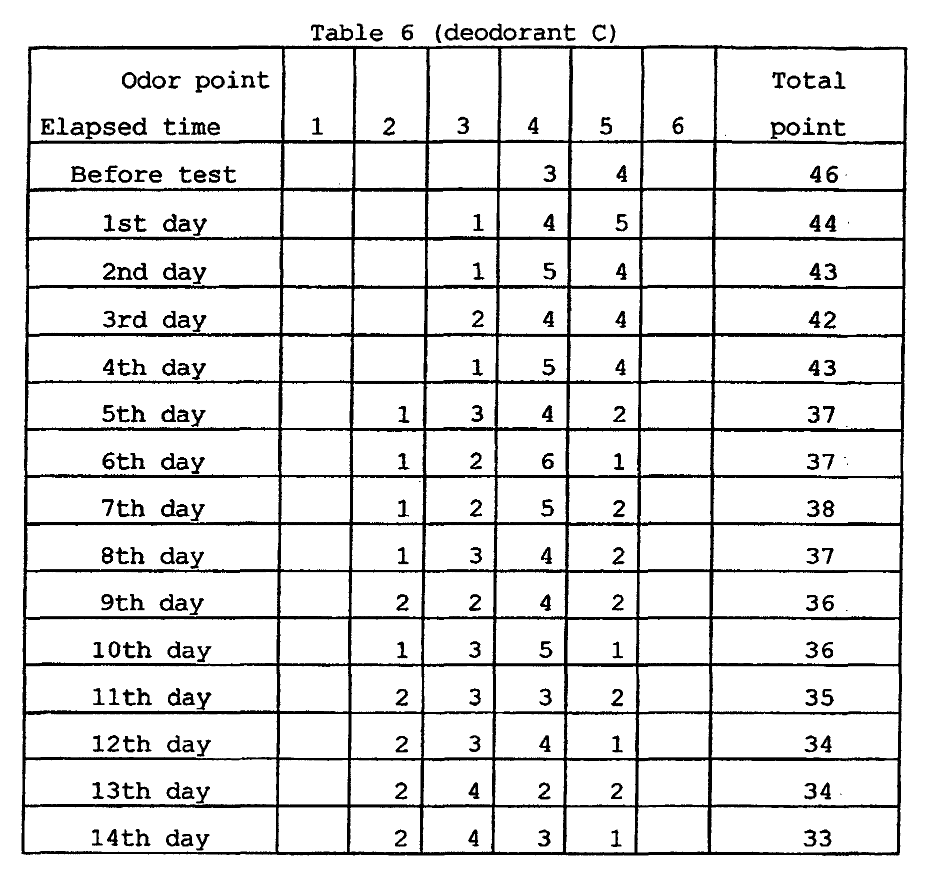

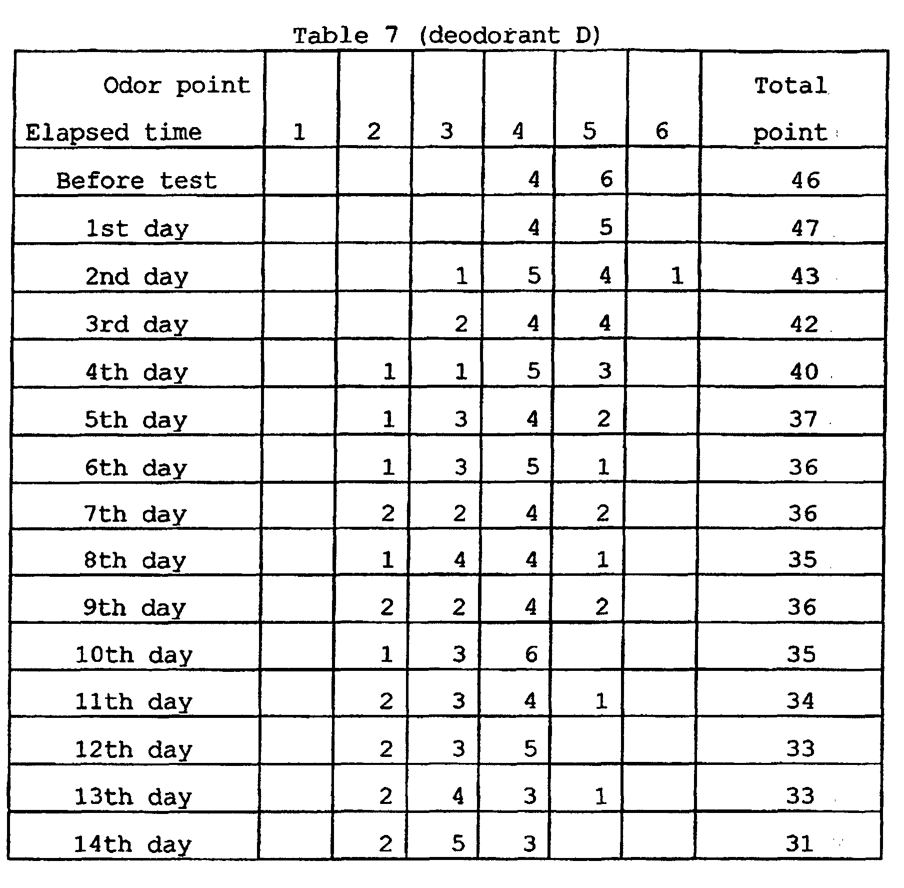

### [2] Effect of Plant-type Feces and Urine Deodorant of the Invention Employing Components (1)-(3)

### (1) Preparation of Deodorants

1) Deodorant E: deodorant C as indicated in [1].
2) Deodorant F: wherein compounding was performed such that 0.98 part by weight of the vaporization residue of the tea leaf extract liquid was contained relative to 100 parts by weight of the vaporization residue of the fermentation liquid in deodorant C.
3) Deodorant G: based on deodorant F, wherein the vaporization residue of the tea leaf extract liquid was present in an amount of 980 parts by weight.

### (2) Formation of Tablets

As in Example 1, tablets of 0.3 g/tablet, containing 0.1 g of the effective deodorizing component per tablet, were formed and used as test samples.

### (3) Method and Result of Test

Student house-living people receiving the same diet were used as subjects. 45 student house-living people were divided into three groups (determined as groups 6)-8)) of 15 people each. The groups are differently treated as follows:
Group 6): took 3 deodorant E-containing tablets/day.
Group 7): took 3 deodorant F-containing tablets/day.
Group 8): took 3 deodorant G-containing tablets/day.

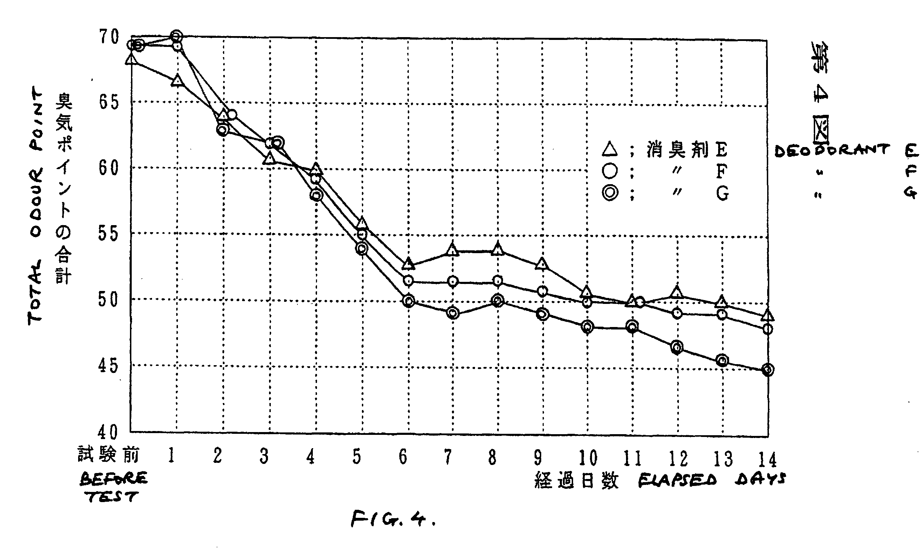

The test was performed for 14 days. In each group, 3 baked garlic pieces of about 5 g each were daily given for 14 consecutive days, in addition to meals. Evaluation of odor was conducted as in Example 1. That is, each person at the test performed sensory evaluation of odor. Based on evaluation references substantially the same as those used in Example 1, points were given. Results are shown in Tables 8-10. The meals given during the period included no food that is likely to produce odor, such as garlic. The student house-living people were given the same contents of meals at the same hours. All the student house-living people were healthy and discharged normal stool before the test and during the test. The total odor points in the results shown in Tables 8-10 are presented in a graph shown in Fig. 4.

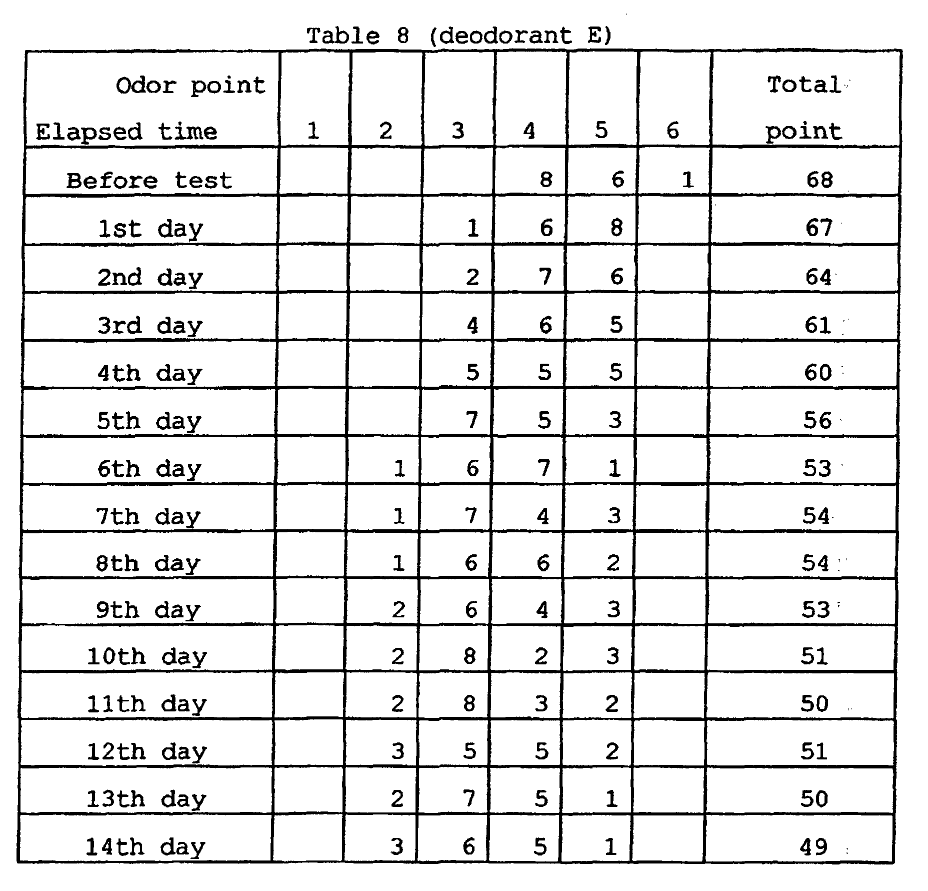

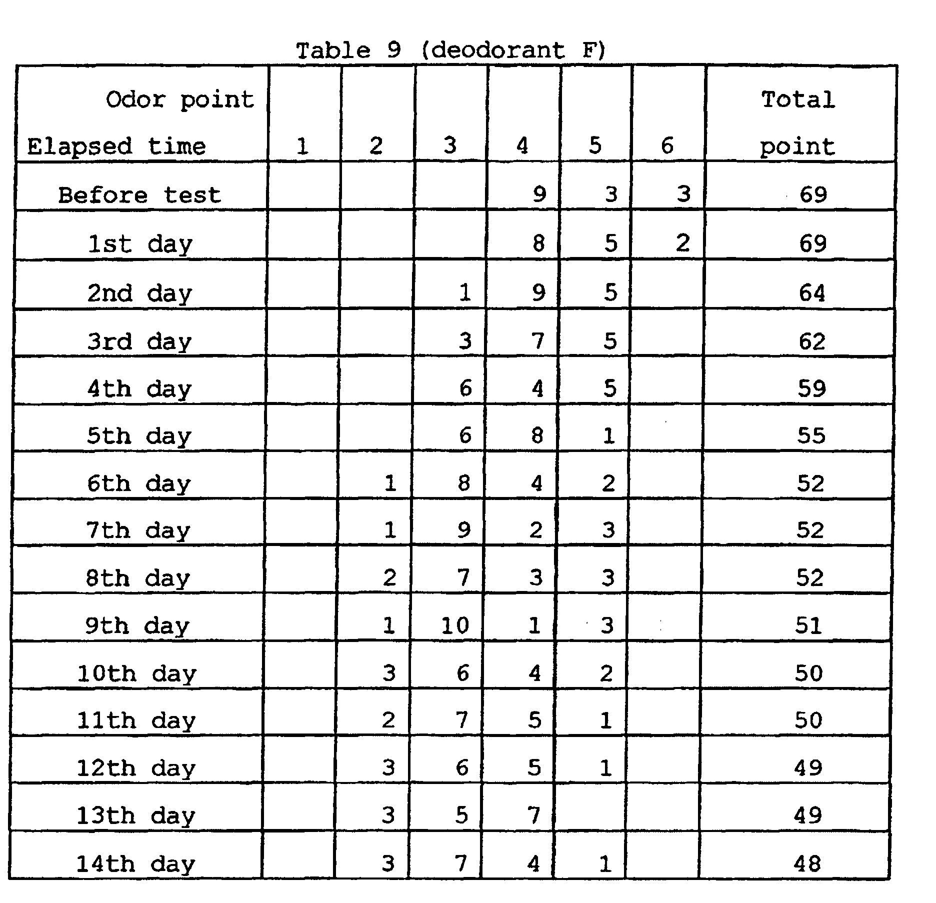

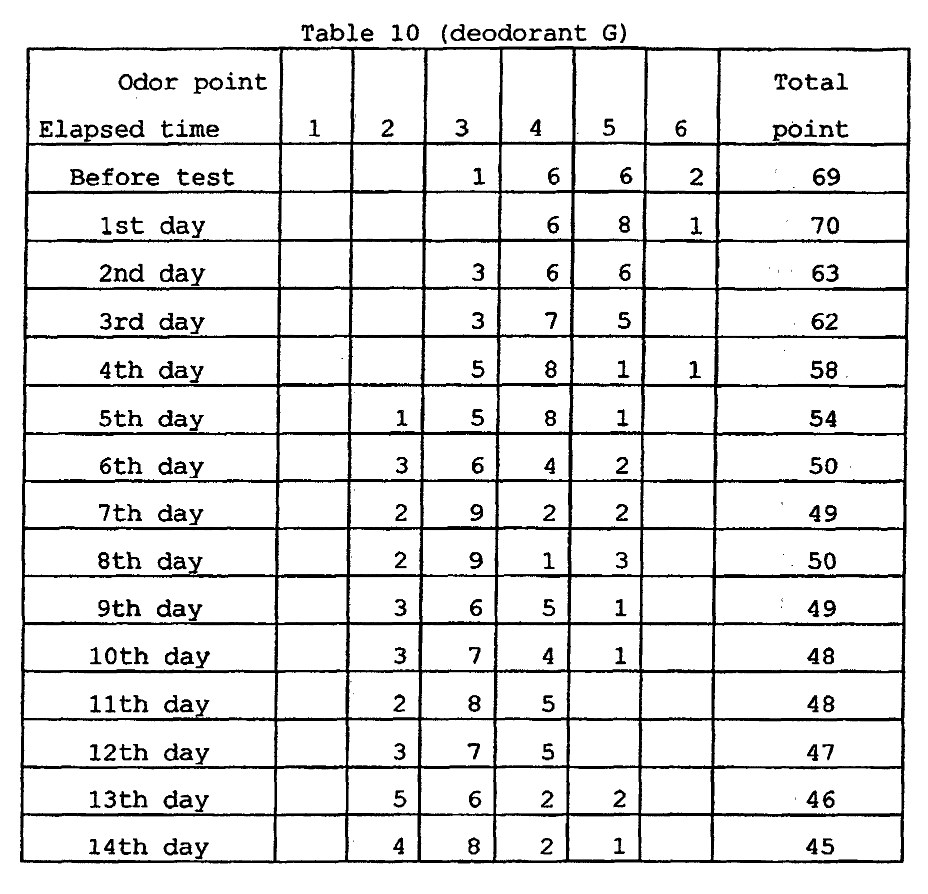

As can be seen from Tables 8-10 and Fig. 4, in the groups that received deodorants E-G, odor reductions started to show on the third to fifth days, and final reductions of about 30-35% in odor strength were observed. In particular, deodorant G, containing a large amount of the vaporization residue of the tea leaf extract liquid, produced a great deodorizing effect, indicating a synergistic effect due to the combination of the tree component extract liquid and the tea leaf extract liquid with the fermentation liquid. Since the deodorizing effect of the tea leaf extract liquid is largely attributed to flavonoid, it is considered that extracts from other tea, such as black tea, oolong tea, rooibos tea, and extracts from hawthorn, fleawort will produce similar effects.

### [Example 3]

In Example 3, the effect of the combined use of the fermentation liquid, i.e., the component (1), and the tree component extract liquid, i.e., the component (2), and a mushroom extract liquid or a carrot leaf extract liquid, i.e., the component (3), in the plant-type feces and urine deodorant of the invention was checked.

### Preparation of Deodorants

1) Deodorant H: having the same composition as that of deodorant C but prepared as a new deodorant.
2) Deodorant I: wherein compounding was performed such that 1 part by weight of the vaporization residue of a mushroom extract liquid was contained relative to 100 parts by weight of the vaporization residue of the fermentation liquid in deodorant H.
3) Deodorant J: based on deodorant I, wherein the vaporization residue of the mushroom extract liquid was present in an amount of 1000 parts by weight.
4) Deodorant K: wherein compounding was performed such that 1 part by weight of the vaporization residue of a carrot leaf extract liquid was contained relative to 100 parts by weight of the vaporization residue of the fermentation liquid in deodorant H.
5) Deodorant L: based on deodorant K, wherein the vaporization residue of the carrot leaf extract liquid was present in an amount of 1000 parts by weight.

The aforementioned mushroom extract liquid employed "Agaricus bisporus" in scientific name as a raw material. The mushroom extract liquid was obtained by extracting the raw material with a solvent containing at least one of water and ethanol while performing heating. The mushroom extract liquid was a brownish liquid having a characteristic aroma and sourness. The aforementioned carrot leaf extract liquid employed a part of carrot leaves, stems or roots or a mixture thereof as a raw material. The carrot leaf extract liquid was obtained by extracting the raw material with a solvent containing at least one of water and ethanol while performing heating. The carrot leaf extract liquid was a brownish liquid having a characteristic aroma.

### (2) Formation of Tablets

As in Example 1, tablets of 0.3 g/tablet, containing 0.1 g of the effective deodorizing component per tablet, were formed and used as test samples.

### (3) Method and Result of Test

Student house-living people receiving the same diet were used as subjects.

50 student house-living people were divided into five groups (determined as groups 1)-5)) of 10 people each. The groups are differently treated as follows:
Group 1): took 3 deodorant H-containing tablets/day.
Group 2): took 3 deodorant I-containing tablets/day.
Group 3): took 3 deodorant J-containing tablets/day.
Group 4): took 3 deodorant K-containing tablets/day.
Group 5): took 3 deodorant L-containing tablets/day.

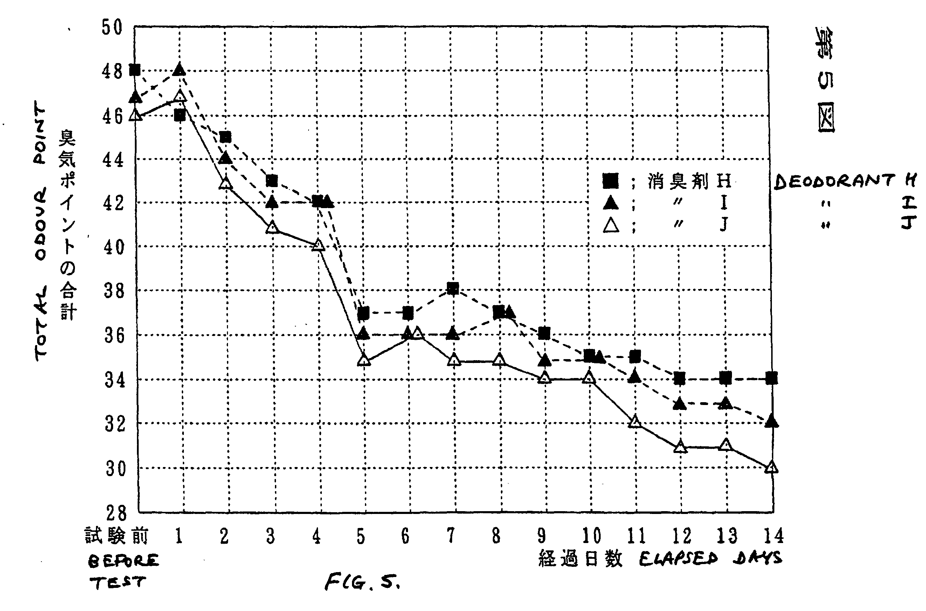

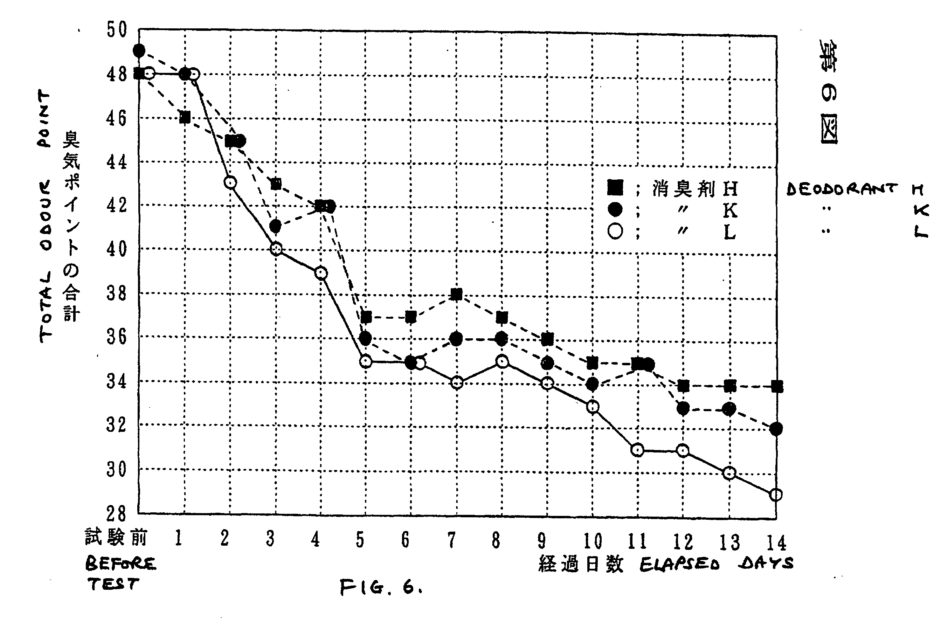

The test was performed for 14 days. In each group, 3 baked garlic pieces of about 5 g each were daily given for 14 consecutive days, in addition to meals. Evaluation of odor was conducted as in Example 1. That is, each person at the test performed sensory evaluation of odor. Based on evaluation references substantially the same as those used in Example 1, points were given. Results are shown in Tables 11-15. The meals given during the period included no food that is likely to produce odor, such as garlic. The student house-living people were given the same contents of meals at the same hours. All the student house-living people were healthy and discharged normal stool before the test and during the test. The total odor points in the results shown in Tables 11-13 are presented in a graph shown in Fig. 5. The total odor points in the results shown in Tables 11, 14 and 15 are presented in a graph shown in Fig. 6.

As can be seen from Tables 11-13 and Fig. 5, in the groups that received deodorants I, J and, in particular, the group that received deodorant J, odor reductions started to show on the third to fifth days, and final reductions of about 30-40% in odor strength were observed. In particular, deodorant J, containing a large amount of the vaporization residue of the mushroom extract liquid, produced a great deodorizing effect, indicating a synergistic effect due to the combination of the tree component extract liquid and the mushroom extract liquid with the fermentation liquid. The same tendency can be seen in Table 11 and Tables 14, 15 and Fig. 6, indicating a synergistic effect due to the combination of the tree component extract liquid and the carrot leaf extract liquid with the fermentation liquid.

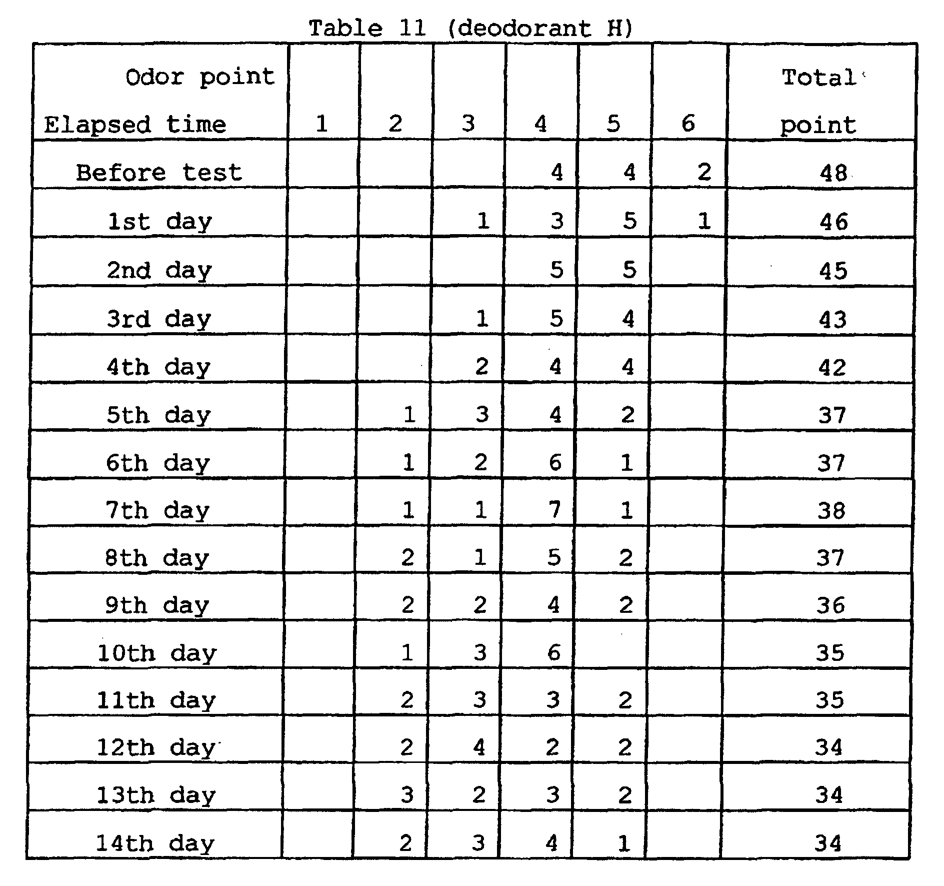

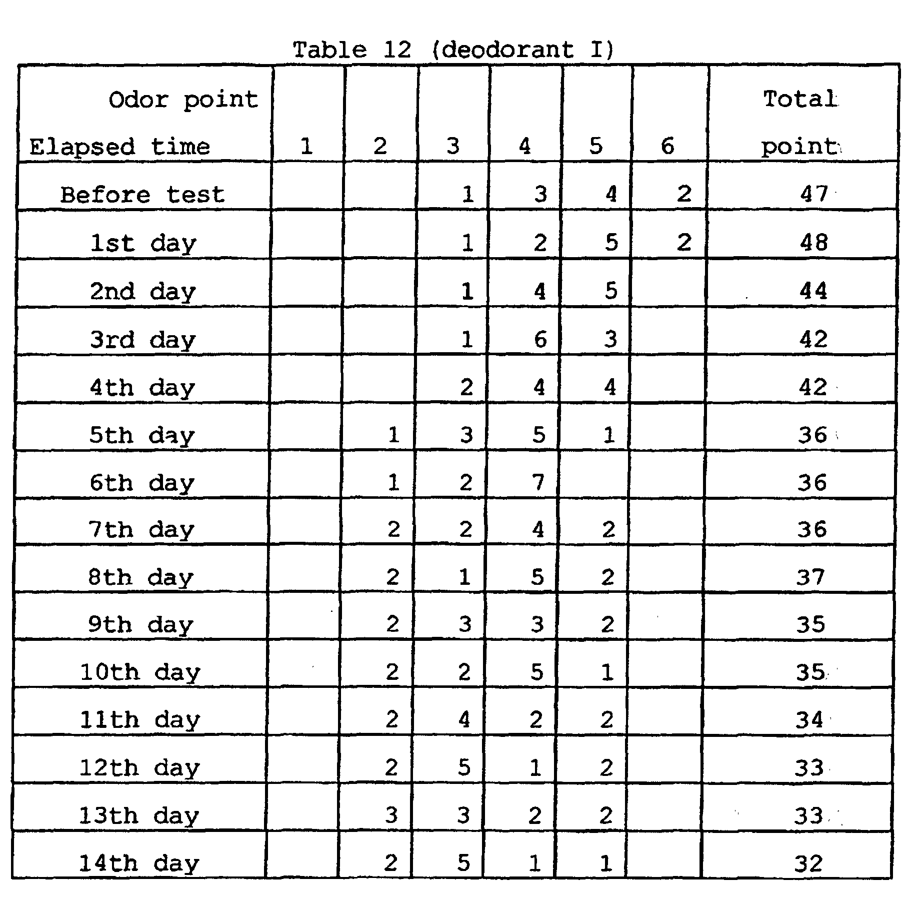

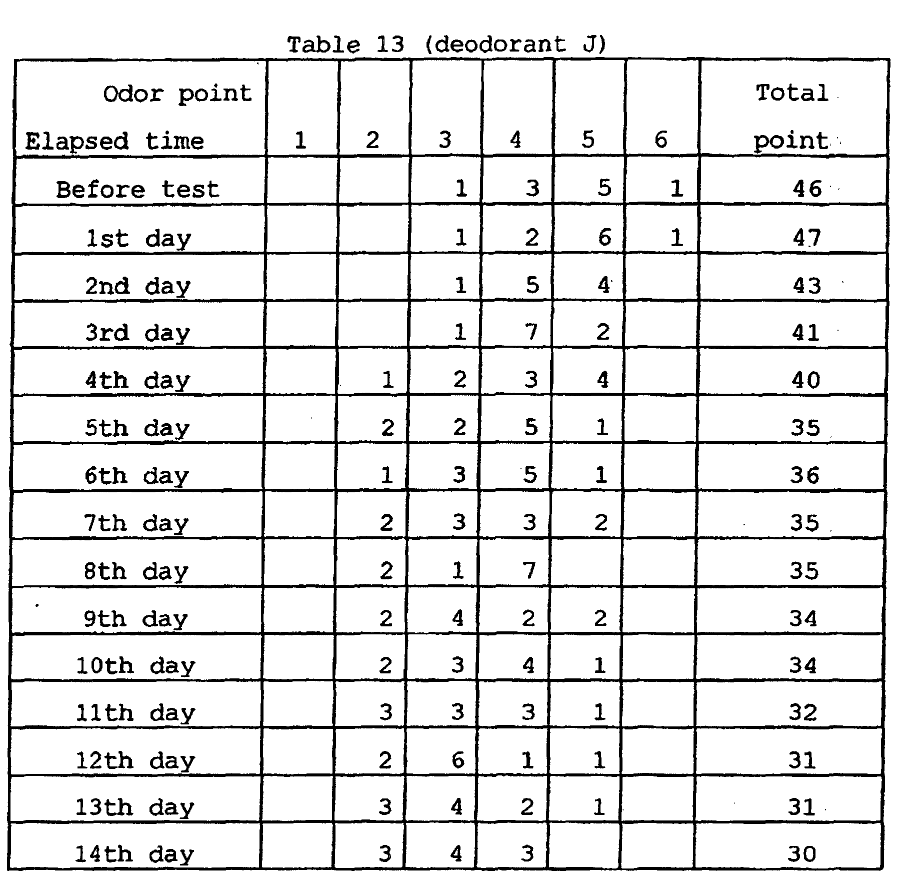

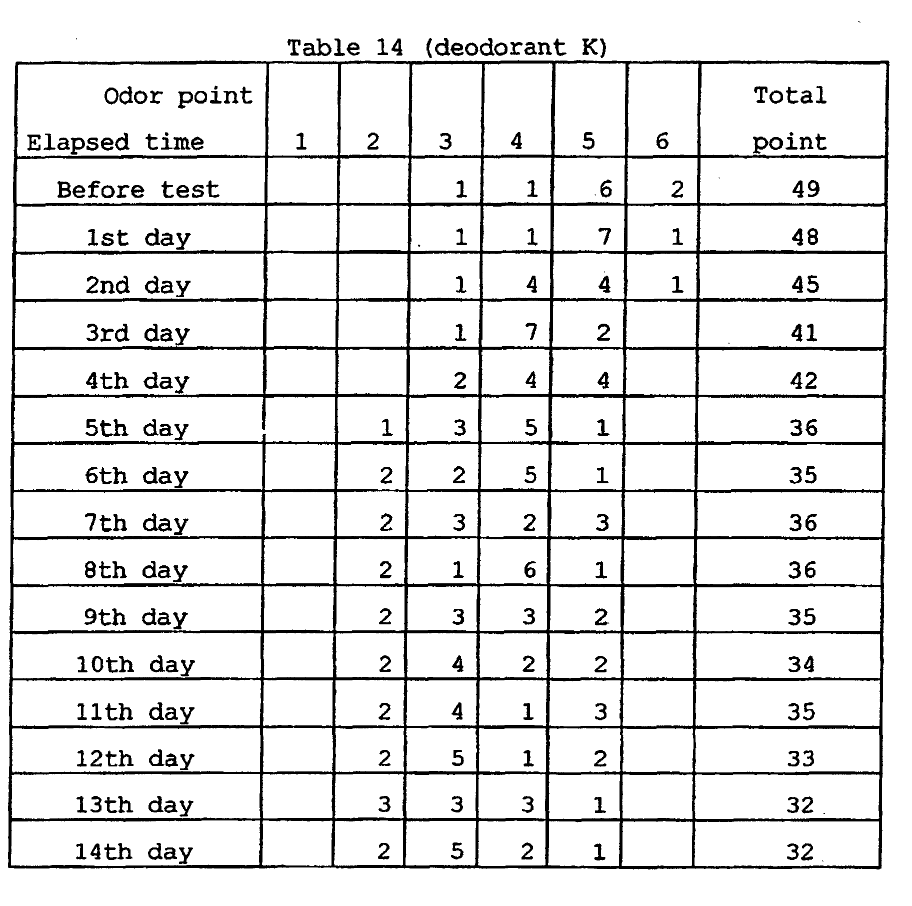

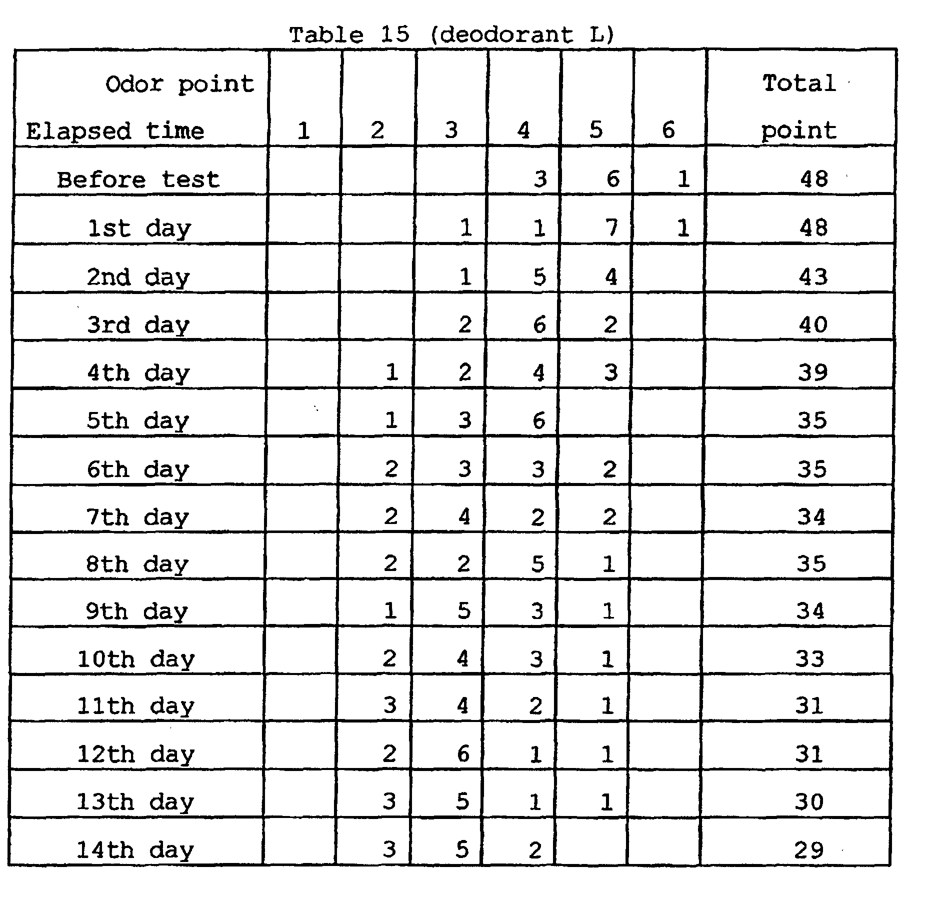

### [Example 4]

In Example 4, the effect of deodorizing dog feces and urine when the plant-type feces and urine deodorant of the invention was orally taken was investigated. In this example, the deodorizing effect was evaluated by measuring the production amounts of hydrogen sulfide, methyl mercaptan and trimethylamine, which are representative odor components contained in stool.

### (1) Preparation of Deodorant Powders

Using deodorant powders A, B and C prepared by the following methods as test samples, test was performed.
1) Deodorant powder A: obtained by powdering the mixture liquid obtained in Example 1 by spray-drying.
2) Deodorant powder B: obtained by preparing a mixture liquid in substantially the same method as in Example 1, except that a mushroom extract liquid was compounded instead of the tea leaf extract liquid, and then powdering the mixture liquid by spray-drying. The mushroom extract liquid was compounded in such an amount that the amount of vaporization residue of the mushroom extract liquid became 200 parts by weight relative to 100 parts by weight of the vaporization residue of the fermentation liquid. The mushroom extract liquid used was the same as the that used in Example 3.
3) Deodorant powder C: obtained by preparing a mixture liquid in substantially the same method as in Example 1, except that a carrot leaf extract liquid was compounded instead of the tea leaf extract liquid, and then powdering the mixture liquid by spray-drying. The carrot leaf extract liquid was compounded in such an amount that the amount of the vaporization residue of the carrot leaf extract liquid became 30 parts by weight relative to 100 parts by weight of the vaporization residue of the fermentation liquid. The carrot leaf extract liquid used was the same as the that used in Example 3.

2) Five dogs (beagles), 5 years old in average age and 10 kg in average body weight, which were diagnosed to be healthy, were used as subjects. Three of them were given deodorant powder A, B or C in an amount of 0.3 g/day once a day for 7 consecutive days in the form of mixture with feed. One dog of the other two dogs was treated as a comparative example and given a sample made of a mixture of green tea and vitamins in an amount of 1.2 g/day for 7 consecutive days, similarly to the three dogs. The other one was treated as a control dog and given only feed. The dogs were allowed to freely drink water. During the period, stool was collected every day, and the odor strengths of the stool samples were measured using a gas detector tube and a smell sensor. All the test samples were completely taken up by the dogs. During the feeding period, all the subject dogs discharged normal stool without any soft or diarrheal stool observed.

### (3) Test Method

The collected stool samples were stored in a frozen state, and evaluated after the test of seven days ended.

In a laboratory room at a room temperature of 25°C and a humidity of 60%, 10 g of each stool sample was weighed out and placed in a glass container having a capacity of 150 mL. Each sample was then left in a dryer at 40°C for about 60 minutes, so that the stool temperature was raised to about 30°C. Using direct reading-type detector tubes (Detector Tube Pump "Model 180" by Gastec Kabushiki Gaisha), the concentrations of hydrogen sulfide (detector tube used: 4LT Hydrogen Sulfide), methyl mercaptan (detector tube used: 70L All Melcaptans) and trimethylamine (detector tube used: 180 Amines) in the glass containers were measured. Furthermore, using a smell sensor ("FRAGRACE SENSOR SF-105" by Sougo Yakko Kabushiki Gaisha), the odor was detected 10 minutes after each sample was taken out of the dryer. The smell sensor used was a sensor that causes odor molecules to adsorb to a synthetic two-molecule coating on a quartz oscillator and measures odor strength based on changes in oscillating frequency (Hz) caused by the adsorption. Higher frequencies indicate stronger odor.

### (4) Test Results

The concentrations of hydrogen sulfide, methyl mercaptan and trimethylamine with respect to the individual subjects are shown in Table 16, Table 17 and Table 18. Measurements obtained by the smell sensor are shown in Table 19. In Tables 16-19, "Control dog" indicates the individual that received only feed, and "Dog a" indicates the individual that received deodorant powder A, and "Dog b" indicates the individual that received deodorant powder B, and "Dog c" indicates the individual that received deodorant powder C, and "Comparative dog" indicates the individual that received the feed made of a mixture of green tea and vitamins.

From Tables 16-18, it can be seen that the dog a, the dog b and the dog c, given deodorant powders A, B and C according to the invention, exhibited reductions of hydrogen sulfide, methyl mercaptan and trimethylamine on the second and later days after the feeding was started, compared with the control dog. This is also apparent from the measurements by the smell sensor shown in Table 19. As can be seen from the comparison between the comparative dog, given the feed made of a mixture of green tea and vitamins, and the dog a, given deodorant powder A according to the invention prepared from a mixture liquid of the predetermined fermentation liquid, the tree component extract liquid and the tea leaf extract liquid, the mixture of green tea and vitamins also produced a deodorizing effect. However, in comparison with the case of the dog a, the effect on the comparative dog was produced in a later stage and only to a small extent.

**Table 16**

| Hydrogen sulfide Unit: ppm | | | | | |
|---|---|---|---|---|---|
| Days of feeding | Control dog | Dog a | Dog b | Dog c | Comparative dog |
| 1 day | 1.1 | 1.0 | 0.6 | 0.9 | 0.9 |
| 2 days | 1.0 | 0.4 | 0.3 | 0.4 | 0.5 |
| 3 days | 0.6 | 0.0 | 0.2 | 0.1 | 0.3 |
| 4 days | 0.6 | 0.0 | 0.0 | 0.0 | 0.5 |
| 5 days | 0.9 | 0.0 | 0.0 | 0.0 | 0.5 |
| 6 days | 1.6 | 0.0 | 0.0 | 0.0 | 0.3 |
| 7 days | 1.1 | 0.0 | 0.0 | 0.0 | 0.3 |

**Table 17**

| Mercaptan Unit: ppm | | | | | |
|---|---|---|---|---|---|
| Days of feeding | Control dog | Dog a | Dog b | Dog c | Comparative dog |
| 1 day | 0.4 | 0.6 | 0.4 | 0.5 | 0.4 |
| 2 days | 0.6 | 0.0 | 0.0 | 0.1 | 0.3 |
| 3 days | 0.6 | 0.0 | 0.0 | 0.0 | 0.3 |
| 4 days | 0.4 | 0.0 | 0.0 | 0.0 | 0.0 |
| 5 days | 0.6 | 0.0 | 0.0 | 0.0 | 0.0 |
| 6 days | 0.6 | 0.0 | 0.0 | 0.0 | 0.2 |
| 7 days | 0.6 | 0.0 | 0.0 | 0.0 | 0.0 |

**Table 18**

| Trimethylamine Unit: ppm | | | | | |
|---|---|---|---|---|---|
| Days of feeding | Control dog | Dog a | Dog b | Dog c | Comparative dog |
| 1 day | 0.0 | 0.0 | 0.0 | 0.0 | 0.0 |
| 2 days | 0.1 | 0.0 | 0.0 | 0.0 | 0.0 |
| 3 days | 0.1 | 0.0 | 0.0 | 0.0 | 0.0 |
| 4 days | 0.2 | 0.0 | 0.0 | 0.0 | 0.0 |
| 5 days | 0.1 | 0.0 | 0.0 | 0.0 | 0.0 |
| 6 days | 0.0 | 0.0 | 0.0 | 0.0 | 0.2 |
| 7 days | 0.2 | 0.0 | 0.0 | 0.0 | 0.0 |

**Table 19**

| Unit: Hz | | | | | |
|---|---|---|---|---|---|
| Days of feeding | Control dog | Dog a | Dog b | Dog c | Comparative dog |
| 1 day | 533 | 525 | 530 | 529 | 523 |
| 2 days | 530 | 483 | 501 | 490 | 508 |
| 3 days | 533 | 460 | 469 | 466 | 482 |
| 4 days | 537 | 452 | 455 | 451 | 473 |
| 5 days | 536 | 440 | 448 | 445 | 475 |
| 6 days | 535 | 435 | 443 | 442 | 463 |
| 7 days | 534 | 433 | 441 | 438 | 476 |

The results indicate that deodorant powder A produced a great deodorizing effect due to the synergetic effect of the predetermined fermentation liquid, the predetermined tree component extract liquid and the predetermined tea leaf extract liquid.

Hydrogen sulfide, methyl mercaptan and trimethylamine, that is, the volatile components treated as measurement objects in Example 4, are said to be produced mainly by intestinal putrefying bacteria. It is speculated that deodorant powders A-C according to the invention produce deodorizing effects through mechanisms, for example, wherein fermentation products promote the growth of intestinal lactic acid bacteria, and wherein the aforementioned volatile components adsorb to fermentation products so that evaporation of the volatile components is restrained, and wherein cyclodextrins in the fermentation products enclose intestinal fat and oil components so that decomposition of intestinal bacteria is restrained. Furthermore, addition of the tree component extract liquid and the plant extract liquid will further enhance the deodorizing effect.

### [Example 5]

In Example 5, with regard to the plant-type feces and urine deodorant of the invention, the urinary concrement reducing effect of mixtures obtained by combined use of the fermentation liquid, i.e., the component (1), and the tree component extract liquid, i.e., the component (2), and the tea leaf extract liquid, i.e., the component (3), was confirmed.

It is said that 70-90% of the cases of urinary concrement of a urologic syndrome are struvite calculus. The composition of calculuses is an aggregate of phosphoric acid, ammonia and magnesium. Therefore, the urinary concrement reducing effect was evaluated using a simplified measurement liquid wherein the magnesium content in urine is measured by a colorimeteric method.

### (1) Preparation of Mixtures

1) Mixture A: wherein compounding was performed such that 2 parts by weight of the vaporization residue (dried solid) of the tree component extract liquid as in Example 1 was contained relative to 100 parts by weight of the vaporization residue (dried solid) of the fermentation liquid as in Example 1.
2) Mixture B: wherein compounding was further performed on the urinary concrement-reducing agent A such that 1 part by weight of the vaporization residue (dried solid) of the tea leaf extract liquid as in Example 1 was contained.
3) Mixture C: wherein compounding was further performed on urinary concrement-reducing agent A such that 1000 parts by weight of the vaporization residue (dried solid) of the tea leaf extract liquid as in Example 1 was contained.

### (2) Test Method

Forty cage-bred cats (26 males and 14 females) were divided into the following four groups. Test was conducted for 28 days.
Group 1): given dry cat food containing 1% by weight of mixture A.
Group 2): given dry cat food containing 1% by weight of mixture B.
Group 3): given dry cat food containing 1% by weight of mixture C.
Group 4): a control group.

### 1) Urine Collecting Method and Urine Testing Method

Urine was collected by a urinary conduction catheter method. The collected urine samples were evaluated with the urinary magnesium content used as an index, using a simplified urinary concrement diagnostic agent ("Proto Stain" in commercial name by Red Heart).

### 2) Method of Measuring Magnesium Content

At the start of the test and at the elapse of 7, 14, 21 and 28 days, 0.1 mL of urine sampled from each cat was injected into 3 mL of the simplified diagnostic agent, and mixed by gentle shaking. After the mixed agent was left at a room temperature for 5 minutes, the color tone was visually determined, and a magnesium content was determined on the basis of the references shown in Table 20 below.

**Table 20**

| Correlation between Color Tone and Magnesium Content | | |
|---|---|---|
| Color tone | Color change index | Magnesium content |
| No color change | 0 | 0 mg/dL |
| Bluish purple | 1 | 1 mg/dL |
| Purple | 2 | 2 mg/dL |
| Reddish purple-Red | 3 | 5 mg/dL or more |

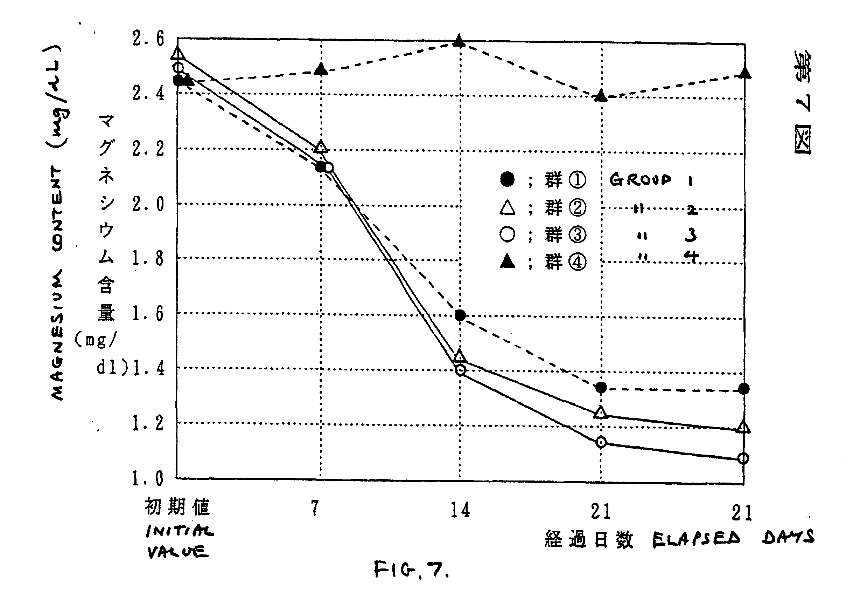

Table 21 shows mean values of 10 cats of each group. Fig. 7 shows a graph wherein the mean values are indicated.

As can be seen from Table 21 and Fig. 7, groups 1)-3), given dry food containing mixtures AS B and C, exhibited considerable magnesium content reductions at the elapse of 7 days and later and, more particularly, at the elapse of 14 days and later, in comparison with group 4), which was given only dry food. This tendency was remarkable in the cases where the tree component extract liquid and the tea leaf extract liquid were used together with the fermentation liquid, and, particularly, in the case where the amount of the vaporization residue of the tea leaf extract liquid was greater, thus confirming the synergistic affect of urinary concrement reduction by the combined use of the three components.

**Table 21**

| Test group | Elapsed days | | | | |
|---|---|---|---|---|---|
| | Initial value | 7 | 14 | 21 | 28 |
| Group 1) | 2.45±0.60 | 2.15±0.67 | 1.60±0.39 | 1.35±0.41 | 1.35±0.34 |
| Group 2) | 2.55±0.06 | 2.20±0.59 | 1.45±0.37 | 1.25±0.35 | 1.20±0.26 |
| Group 3) | 2.50±0.53 | 2.15±0.67 | 1.40±0.39 | 1.15±0.41 | 1.10±0.32 |
| Group 4) | 2.45±0.60 | 2.50±0.71 | 2.60±0.52 | 2.40±0.66 | 2.50±0.53 |

Although, in deodorant powder A used in Example 4, the fermentation liquid, the tree component extract liquid and the tea leaf (green tea) extract liquid were compounded so that 2 parts by weight of the vaporization residue of the tree component extract liquid and 60 parts by weight of the vaporization residue of the green tea extract liquid were present relative to 100 parts by weight of the vaporization residue of the fermentation liquid, the following modifications are also possible.

Deodorants obtained by powdering after the following extract liquids have been compounded in place of the green tea extract liquid also produce deodorizing effects that are equivalent to or greater than that of Example 4:
(1) vaporization residues of a green tea extract liquid and a hawthorn extract liquid each in an amount of 30 parts by weight;
(2) vaporization residues of a green tea extract liquid, a hawthorn extract liquid and a rooibos tea extract liquid each in an amount of 20 parts by weight;
(3) a vaporization residue of a green tea extract liquid in an amount of 20 parts by weight;
(4) vaporization residues of a green tea extract liquid, a hawthorn extract liquid, a rooibos tea extract liquid and a parsley extract liquid each in an amount of 20 parts by weight; or
(5) vaporization residues of a green tea extract liquid, a hawthorn extract liquid, a rooibos tea extract liquid, a parsley extract liquid and a celery extract liquid each in an amount of 20 parts by weight,

### INDUSTRIAL APPLICABILITY

As described above, the plant-type feces and urine deodorant of the invention has an excellent deodorizing effect on human and animal feces and urine. Therefore, despite keeping a pet, such as a cat, indoors, an event that a room is filled with odor will not occur. Furthermore, in fields of geriatric medical care, patient nursing, the deodorant of the invention will reduce the feces and urine odor of patients who use diapers, so that the loads on caregivers, who normally have to exercise their patience, can be reduced. Further, young women tend to be so much concerned about the odor of their own feces that they hold defecation desire outside their homes. This tendency is a factor that causes constipation. Since use of the plant-type feces and urine deodorant of the invention reduces feces odor, the invention achieves advantages in prevention of constipation and maintenance of health and beauty.

Since the plant-type feces and urine deodorant of the invention produces the deodorizing effect, for example, when it is taken by a living body, the deodorant of the invention prevents production of feces odor before defecation. Due to reduced volatilization and diffusion of odor components into the atmosphere, an excellent deodorizing effect is achieved. With regard to use for animals, even if cats do not defecate or urinate in a predetermined place, the plant-type feces and urine deodorant of the invention achieves deodorization, unlike the conventional deodorants. Further, it becomes unnecessary to use cat litter, which are difficult to handle.

Furthermore, since the deodorant of the invention is made of natural fermentation substances from completely natural materials, the deodorant is safe to living bodies. In addition, since rice bran, which is useful as a health food, is used as a raw material, the deodorant has good nutrition balance. Further, since the deodorant of the invention employs a fermentation liquid and extract liquids, the deodorant has high concentrations of effective components and excellent quality, in comparison with deodorants wherein plants are directly used. Further, for use to animals, the deodorant of the invention can easily be mixed into pet food.

## Claims

1. A plant-type feces and urine deodorant containing (1) a fermentation liquid or a vaporization residue thereof, the fermentation liquid being produced by inoculating Bacillus subtilis or Bacillus natto into a liquid medium which contains rice bran, soybean, a carbon source and water, and which has been adjusted to pH 7.5-10 by an alkaline agent, and by culturing it and filtering it, (2) a tree component extract liquid made using at least one of cedar, cypress and pine as a raw material, or a vaporization residue of the tree component extract liquid, and (3) at least one selected from a tea leaf extract liquid or a vaporization residue thereof, an Umbelliferae plant extract liquid or a vaporization residue thereof, or a mushroom extract liquid or a vaporization residue thereof; and having an effect of reducing odor of feces and urine through oral administration.

2. A plant-type feces and urine deodorant according to claim 1, wherein the amount of the vaporization residue of the tree component extract liquid is 0.001-0.2 part by weight relative to 1 part by weight of the vaporization residue of the fermentation liquid.

3. A plant-type feces and urine deodorant according to claim 1 or 2, wherein the tea leaf extract liquid or the vaporization residue thereof is contained, and the amount of the vaporization residue thereof is 10-1000 parts by weight relative to 100 parts by weight of the vaporization residue of the fermentation liquid.

4. A plant-type feces and urine deodorant according to claim 1 or 2, wherein the amount of the vaporization residue of the mushroom extract liquid and/or the Umbelliferae plant extract liquid is 10-1000 parts by weight relative to 100 parts by weight of the vaporization residue of the tea leaf extract liquid, and the total amount of the vaporization residue of the mushroom extract liquid and/or the Umbelliferae plant extract liquid and the vaporization residue of the tea leaf extract liquid is 10-1000 parts by weight relative to 100 parts by weight of the vaporization residue of the fermentation liquid.

5. A plant-type feces and urine deodorant containing (1) a fermentation liquid or a vaporization residue thereof, the fermentation liquid being produced by inoculating Bacillus subtilis or Bacillus natto into a liquid medium which contains rice bran, soybean, a carbon source and water, and which has been adjusted to pH 7.5-10 by an alkaline agent, and by culturing it and filtering it, (2) a tree component extract liquid made using at least one of cedar, cypress, pine as a raw material, or a vaporization residue of the tree component extract liquid, and (3) at least one selected from a tea leaf extract liquid or a vaporization residue thereof, an Umbelliferae plant extract liquid or a vaporization residue thereof, or a mushroom extract liquid or a vaporization residue thereof; and having an effect of reducing odor of feces and urine and an effect of reducing urinary concrement through oral administration.

## Patentansprüche

1. Pflanzliches Desodorant für Stuhl und Urin, mit (1) einer Fermentationsflüssigkeit oder einem Verdampfungsrückstand davon, wobei die Fermentationsflüssigkeit durch Einimpfen von Bacillus subtilis oder Bacillus natto in ein flüssiges Medium, welches Reiskleie, Sojabohnen, eine Kohlenstoffquelle und Wasser enthält und welches mit einem alkalischen Mittel auf pH 7.5 bis 10 eingestellt worden ist, und durch Kultivieren und Filtrieren hergestellt wird, (2) einem unter Verwendung zumindest einer der Pflanzen Zeder, Zypresse und Pinie als ein Rohmaterial hergestelltes flüssiges Baumbestandteilextrakt oder einem Verdampfungsrückstand des flüssigen Baumbestandteilextraktes und (3) mit zumindest einem Stoff, ausgewählt aus einem flüssigen Teeblattextrakt oder einem Verdampfungsrückstand davon, einem flüssigen Doldenblütlerextrakt oder einem Verdampfungsrückstand davon oder einem flüssigen Pilzextrakt oder einem Verdampfungsrückstand davon; und mit einer Wirkung, durch orale Verabreichung Geruch von Stuhl und Urin zu reduzieren.

2. Pflanzliches Desodorant für Stuhl und Urin gemäß Anspruch 1, wobei die Menge des Verdampfungsrückstandes des flüssigen Baumbestandteilexktrates 0,001 bis 0,2 Gewichtsanteile beträgt, bezogen auf 1 Gewichtsanteil des Verdampfungsrückstandes der Fermentationsflüssigkeit.

3. Pflanzliches Desodorant für Stuhl und Urin nach Anspruch 1 oder 2, in welchem das flüssige Teeblattextrakt oder der Verdampfungsrückstand davon enthalten ist und bei dem die Menge des Verdampfungsrückstandes davon 10 bis 1000 Gewichtsanteile beträgt, bezogen auf 100 Gewichtsanteile des Verdampfungsrückstandes der Fermentationsflüssigkeit.

4. Pflanzliches Desodorant für Stuhl und Urin nach Anspruch 1 oder 2, wobei die Menge des Verdampfungsrückstandes des flüssigen Pilzextraktes und/oder des flüssigen Doldenblütlerextraktes 10 bis 1000 Gewichtsanteile beträgt, bezogen auf 100 Gewichtsanteile des Verdampfungsrückstandes des flüssigen Teeblattextraktes und wobei die Gesamtmenge des Verdampfungsrückstandes des flüssigen Pilzextraktes und/oder des flüssigen Doldenblütlerextraktes und der Verdampfungsrückstand des flüssigen Teeblattextraktes 10 - 1000 Gewichtsanteile beträgt, bezogen auf 100 Gewichtsanteile des Verdampfungsrückstandes der Fermentationsflüssigkeit.

5. Pflanzliches Desodorant für Stuhl und Urin, mit (1) einer Fermentationsflüssigkeit oder einem Verdampfungsrückstand davon, wobei die Fermentationsflüssigkeit durch Einimpfen von Bacillus subtilis oder Bacillus natto in ein flüssiges Medium, welches Reiskleie, Sojabohnen, eine Kohlenstoffquelle und Wasser enthält und welches mit einem alkalischen Mittel auf pH 7.5 bis 10 eingestellt worden ist, und durch Kultivieren und Filtrieren hergestellt wird, (2) einem unter Verwendung zumindest einer der Pflanzen Zeder, Zypresse und Pinie als ein Rohmaterial hergestelltes flüssiges Baumbestandteilextrakt oder einem Verdampfungsrückstand des flüssigen Baumbestandteilextraktes und (3) mit zumindest einem Stoff, ausgewählt aus einem flüssigen Teeblattextrakt oder einem Verdampfungsrückstand davon, einem flüssigen Doldenblütlerextrakt oder einem Verdampfungsrückstand davon oder einem flüssigen Pilzextrakt oder einem Verdampfungsrückstand davon; und mit einer Wirkung, durch orale Verabreichung Geruch von Stuhl und Urin zu reduzieren und ferner Harnstein zu reduzieren.

## Revendications

1. Désodorisant de type végétal pour les matières fécales et l'urine, contenant (1) un liquide de fermentation ou un résidu d'évaporation de celui-ci, le liquide de fermentation étant produit par l'inoculation d'un Bacillus subtilis ou Bacillus natto dans un milieu liquide qui contient du son de riz, des graines de soja, une source de carbone et de l'eau, et qui a été ajusté à un pH de 7,5-10 par un agent alcalin, et par la mise en culture et la filtration de celui-ci, (2) un liquide extrait d'un composant d'arbre obtenu par l'utilisation d'au moins l'un parmi le cèdre, le cyprès et le pin comme matière première, ou un résidu d'évaporation du liquide extrait d'un composant d'arbre, et (3) au moins un choisi parmi un liquide extrait d'une feuille de thé ou un résidu d'évaporation de celui-ci, un liquide extrait d'une plante ombellifère ou un résidu d'évaporation de celui-ci, ou un liquide extrait de champignon ou un résidu d'évaporation de celui-ci; et ayant pour effet de réduire l'odeur des matières fécales et de l'urine par administration orale.

2. Désodorisant de type végétal pour les matières fécales et l'urine selon la revendication 1, dans lequel la quantité de résidu d'évaporation du liquide extrait d'un composant d'arbre est de 0,001-0,2 partie en poids par rapport à 1 partie en poids du résidu d'évaporation du liquide de fermentation.

3. Désodorisant de type végétal pour les matières fécales et l'urine selon la revendication 1 ou 2, dans lequel le liquide extrait d'une feuille de thé ou le résidu d'évaporation de celui-ci est contenu, et la quantité de résidu d'évaporation de celui-ci est de 10-1000 parties en poids par rapport à 100 parties en poids du résidu d'évaporation du liquide de fermentation.

4. Désodorisant de type végétal pour les matières fécales et l'urine selon la revendication 1 ou 2, dans lequel la quantité de résidu d'évaporation du liquide extrait du champignon et/ou de liquide extrait d'une plante ombellifère est de 10-1000 parties en poids par rapport à 100 parties en poids du résidu d'évaporation du liquide extrait d'une feuille de thé, et la quantité totale de résidu d'évaporation du liquide extrait du champignon et/ou de liquide extrait d'une plante ombellifère et le résidu d'évaporation du liquide extrait d'une feuille de thé est de 10-1000 parties en poids par rapport à 100 parties en poids du résidu d'évaporation du liquide de fermentation.

5. Désodorisant de type végétal pour les matières fécales et l'urine contenant (1) un liquide de fermentation ou un résidu d'évaporation de celui-ci, le liquide de fermentation étant produit par inoculation d'un Bacillus subtilis ou Bacillus natto dans un milieu liquide qui contient du son de riz, des graines de soja, une source de carbone et de l'eau, et qui a été ajusté à un pH de 7,5-10 par un agent alcalin, et par la mise en culture et la filtration de celui-ci, (2) un liquide extrait d'un composant d'arbre obtenu par l'utilisation d'au moins l'un parmi le cèdre, le cyprès et le pin comme matière première, ou un résidu d'évaporation du liquide extrait d'un composant d'arbre, et (3) au moins un choisi parmi un liquide extrait d'une feuille de thé ou un résidu d'évaporation de celui-ci, un liquide extrait d'une plante ombellifère ou un résidu d'évaporation de celui-ci, ou un liquide extrait de champignon ou un résidu d'évaporation de celui-ci; et ayant pour effet de réduire l'odeur des matières fécales et de l'urine et pour effet de réduire les concrétions urinaires par administration orale.
